(19) Europäisches Patentamt
European Patent Office
Office européen des brevets

(11) EP 3 778 585 B1

# (12) EUROPEAN PATENT SPECIFICATION

(45) Date of publication and mention of the grant of the patent:
**19.10.2022 Bulletin 2022/42**

(21) Application number: **19777026.6**

(22) Date of filing: **29.03.2019**

(51) International Patent Classification (IPC):
***C07D 401/12*** (2006.01) ***C07D 403/12*** (2006.01)
***C07D 215/233*** (2006.01) ***A61K 31/4709*** (2006.01)
***A61P 35/00*** (2006.01)

(52) Cooperative Patent Classification (CPC):
**A61P 35/00; A61K 31/4709; C07D 215/233; C07D 401/12; C07D 403/12**

(86) International application number:
**PCT/CN2019/080659**

(87) International publication number:
**WO 2019/185064 (03.10.2019 Gazette 2019/40)**

## (54) QUINOLINE OR QUINAZOLINE COMPOUND AND APPLICATION THEREOF

CHINOLIN- ODER CHINAZOLINVERBINDUNG UND IHRE VERWENDUNG

COMPOSÉ DE QUINOLÉINE OU DE QUINAZOLINE ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2018 CN 201810277244**

(43) Date of publication of application:
**17.02.2021 Bulletin 2021/07**

(73) Proprietors:
- **Haihe Biopharma Co., Ltd.
Shanghai 201203 (CN)**
- **Jinan University
Tianhe District
Guangzhou
Guangdong 510632 (CN)**
- **Shanghai Institute of Materia Medica, Chinese Academy of Sciences
Pudong, Shanghai 201203 (CN)**

(72) Inventors:
- **DING, Ke**
**Guangdong 510632 (CN)**
- **GENG, Meiyu**
**Shanghai 201203 (CN)**
- **CHAN, Shingpan**
**Guangdong 510632 (CN)**
- **DING, Jian**
**Shanghai 201203 (CN)**
- **TAN, Li**
**Guangdong 510632 (CN)**
- **AI, Jing**
**Shanghai 201203 (CN)**
- **ZHANG, Zhang**
**Guangdong 510632 (CN)**
- **PENG, Xia**
**Shanghai 201203 (CN)**
- **REN, Xiaomei**
**Guangdong 510632 (CN)**
- **JI, Yinchun**
**Shanghai 201203 (CN)**
- **TU, Zhengchao**
**Guangdong 510632 (CN)**
- **DAI, Yang**
**Shanghai 201203 (CN)**
- **LU, Xiaoyun**
**Guangdong 510632 (CN)**

(74) Representative: **Heller, Benjamin Henry et al
Kilburn & Strode LLP
Lacon London
84 Theobalds Road
London WC1X 8NL (GB)**

(56) References cited:
**WO-A1-2017/028797 CN-A- 102 643 268
CN-A- 107 151 240**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LI TAN ET AL: "4-Oxo-1,4-dihydroquinoline-3-carboxamide Derivatives as New Axl Kinase Inhibitors", JOURNAL OF MEDICINAL CHEMISTRY, vol. 59, no. 14, 28 July 2016 (2016-07-28) , pages 6807-6825, XP055365082, ISSN: 0022-2623, DOI: 10.1021/acs.jmedchem.6b00608**

## Description

### TECHNICAL FIELD

The present disclosure relates to the field of chemical medicine technology, particularly to a quinoline or quinazoline compound and applications thereof.

### BACKGROUND

AXL is a class of receptor tyrosine kinases and belongs to a TAM receptor tyrosine kinase family that also includes two other members: Mer and Tyro3. TAM was first found in tumor cells, of which the overexpression and ectopic expression are closely related to immunoregulation, and tumor proliferation, growth and migration. AXL was isolated from patients with chronic myeloid leukemia and patients with chronic myeloproliferative disease in 1988. AXL is widely expressed in brain, immune cells, platelets, endothelial cells, skeletal muscle, heart, liver, kidney, and other tissues. Vitamin K-dependent protein kinase Gas6 (growth arrest-specific 6) is the most widely studied AXL ligand currently found, and other ligands in the TAM family include Protein S, Tubby, Tulp-1, and Galectin-3. The TAM family has a similar protein structure, which is mainly composed of three parts: a extracellular domain, a transmembrane domain and a intracellular domain. The extracellular domain comprises two N-terminal immunoglobulin-like regions Ig, and two fibronectin III repeat fragments (FNIII) . The Gas6, after combined with the extracellular domain of AXL, induces the dimerization of AXL, initiating trans-autophosphorylation of the intracellular domain, thereby activating the intracellular signaling pathway and regulating a series of physiological activities, such as, regulating the growth and proliferation of cells through a Src/MAPK/ERK pathway, stimulating the expression of anti-apoptotic proteins through a PI3K/AKT pathway, regulating the migration and proliferation of cells through a PI3K/p38/MAPK pathway. In addition to the Gas6-dependent activation, AXL may be activated in a ligand-independent manner. AXL is involved in the adhesion and immunoregulation action of normal cells. Studies have found that overexpression of AXL exists in a variety of tumor cells, and the signaling pathway regulated by Gas6/AXL is closely related to the occurrence and development of various tumors, such as chronic myeloid leukemia, breast cancer, prostate cancer, non-small cell lung cancer, pancreatic cancer, melanoma, glioma, and renal cell carcinoma. It has been confirmed that inhibiting the expression of AXL can reduce the proliferation and growth of pancreatic cancer cells and inhibit the invasion and migration of breast cancer cells. In non-small cell lung cancer, gene silencing of AXL can inhibit the growth of a tumor. At the same time, the high expression of AXL is also related to the recurrence of a tumor and the tolerance of other anti-cancer drugs, such as imatinib (Gliver), erlotinib (Tarceva), and lapatinib (Tyverb). These evidences indicate that AXL is an effective target of tumor targeting therapy.

Although Bosutinib (SKI606, PF5208763, Bosulif; Pfizer, 2012), Cabozantinib (XL184, Cometriq; Exelixis, 2012), Sunitinib (SU11248, Sutent; Pfizer, 2006) and other marketed drugs have AXL activity, they are multi-targeted drugs without specific. BGB324 (R428; Rigel Pharmaceuticals, BergenBio) is currently known as the most specific small molecule inhibitor of AXL, which is in phase II clinical research and was awarded the title of "Orphan drug for AML treatment" by the FDA in December 2014. At present, there are no small molecule inhibitors directing against AXL kinases on the market. Further reference is made to WO2017/028797.

### SUMMARY

Based on this, the present disclosure provides quinoline or quinazoline compounds, which have a good inhibitory activity of AXL kinase and has an advantage of good metabolic stability.

The specific technical solutions are as follows:

A quinoline or quinazoline compound having a structure represented by a formula (I) or a pharmaceutically acceptable salt thereof, a stereoisomer thereof, or a deuterated analog thereof:

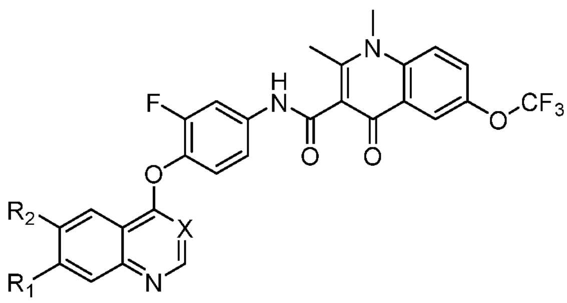

( I )

wherein, X is selected from: CH and N;

$R_1$ and $R_2$ are each independently selected from the group consisting of: hydrogen, halogen, $-(CR_4R_5)_OR_3$ and $-O(CR_4R_5)_OR_3$;
wherein, o is an integer from 0 to 6;
$R_3$, $R_4$, $R_5$ are each independently selected from a group consisting of: -H, $C_1$~$C_6$ alkyl, halogen, $-CF_3$, $-OCF_3$, $-(C=O)-NR_8R_9$, $-COOR_8$, $-SOm-NR_8R_9$, $-CHR_8R_9$, $-OR_8$ and $-NR_8R_9$;
$R_8$ and $R_9$ are each independently selected from: hydrogen, halogen and $C_1$~$C_6$ alkyl, or, $R_8$ and $R_9$, together with N connected thereto, form a saturated or unsaturated 5- to 8-membered heterocyclic group; wherein, the saturated or unsaturated 5- to 8-membered heterocyclic group may be independently and optionally substituted with one or more $R_{10}$; wherein $R_{10}$ is $C_1$~$C_6$ alkyl;
or, $R_1$ and $R_2$ form a substituted or unsubstituted $C_5$~$C_{18}$ aliphatic cycloalkyl containing 1 to 4 heteroatoms.

In some of these embodiments, $R_1$, $R_2$ are each independently $-O(CR_4R_5)_OR_3$;

$R_3$, $R_4$, $R_5$ are each independently selected from a group consisting of: -H, $C_1$~$C_6$ alkyl, $-OR_8$ and $-NR_8R_9$;
$R_8$ and $R_9$ are each independently $C_1$~$C_6$ alkyl, or, $R_8$ and $R_9$, together with N connected thereto, form a saturated or unsaturated 5- to 8-membered heterocyclic group; wherein, the saturated or unsaturated 5- to 8-membered heterocyclic group may be independently and optionally substituted with one or more $R_{10}$; wherein $R_{10}$ is $C_1$~$C_6$ alkyl.

In some of these embodiments, $R_1$ is $-O(CH_2)_OR_3$;

o is an integer from 0 to 4;
$R_3$ is selected from a group consisting of: -H, $C_1$~$C_6$ alkyl, $C_1$~$C_3$ alkoxy and $-NR_8R_9$;
$R_8$ and $R_9$ are each independently $C_1$~$C_3$ alkyl, or, $R_8$ and $R_9$, together with N connected thereto, form a saturated or unsaturated 5- to 6-membered heterocyclic group; wherein, the saturated or unsaturated 5- to 6-membered heterocyclic group may be independently and optionally substituted with one or more $R_{10}$; wherein $R_{10}$ is $C_1$~$C_3$ alkyl.

In some of these embodiments, $R_1$ is selected from a group consisting of: methoxyl, ethoxyl, propoxyl, 2-methoxyethoxyl, 3-methoxypropoxyl, 3-morpholinopropoxyl, 2-(pyrrolidin-1-yl) ethoxyl, 3-(pyrrolidin-1-yl) propoxyl, (piperidin-1-yl) ethoxyl, (piperidin-1-yl) propoxyl, 4-methoxybutoxyl, 2-morpholinoethoxyl, (4-methylpiperazin-1-yl) propoxyl, dimethylaminoethoxyl and isopentyloxyl.
In some of these embodiments, $R_2$ is $-O(CH_2)_OR_3$;

o is an integer from 0 to 4;
$R_3$ is selected from a group consisting of: -H, $C_1$~$C_3$ alkyl, $C_1$~$C_3$ alkoxy and $-NR_8R_9$;
or, $R_8$ and $R_9$, together with N connected thereto, form a saturated 5- to 6-membered heterocyclic group.

In some of these embodiments, $R_2$ is selected from a group consisting of: methoxyl, ethoxyl, propoxyl, 2-methoxyethoxyl, 3-methoxypropoxyl, 2-morpholinoethoxyl and 3-morpholinopropoxyl.
In some of these embodiments, X is N.
In some of these embodiments, the quinoline or quinazoline compound is selected from a group consisting of:

*N*-(3-fluoro-4-((6-methoxy-7-(3-morpholinopropoxy)quinazolin-4-yl)oxy)phenyl)-1,2-dimethy l-4-oxo-6-(trifluoromethoxy)-1,4-dihydroquinoline-3-carboxamide,

*N*-(4-((6,7-bis(2-methoxyethoxy)quinazolin-4-yl)oxy)-3-fluorophenyl)-1,2-dimethyl-4-oxo-6-( trifluoromethoxy)-1,4-dihydroquinoline-3-carboxamide,
*N*-(4-((6,7-dimethoxyquinazolin-4-yl)oxy)-3-fluorophenyl)-1,2-dimethyl-4-oxo-6-(trifluorome thoxy)-1,4-dihydroquinoline-3-carboxamide,
*N*-(3-fluoro-4-((7-methoxy-6-(3-methoxypropoxy)quinazolin-4-yl)oxy)phenyl)-1,2-dimethyl-4 -oxo-6-(trifluoromethoxy)-1,4-dihydroquinoline-3-carboxamide,
*N*-(3-fluoro-4-((6-methoxy-7-(4-methoxybutoxy)quinazolin-4-yl)oxy)phenyl)-1,2-dimethyl-4-oxo-6-(trifluoromethoxy)-1,4-dihydroquinoline-3-carboxamide,
*N*-(3-fluoro-4-((6-methoxy-7-(2-morpholinoethoxy)quinazolin-4-yl)oxy)phenyl)-1,2-dimethyl-4-oxo-6-(trifluoromethoxy)-1,4-dihydroquinoline-3-carboxamide,
*N*-(3-fluoro-4-((6-methoxy-7-(3-(pyrrolidin-1-yl)propoxy)quinazolin-4-yl)oxy)phenyl)-1,2-di methyl-4-oxo-6-(trifluoromethoxy)-l,4-dihydroquinoline-3-carboxamide,
*N*-(3-fluoro-4-((6-methoxy-7-(2-(piperidin-1-yl)ethoxy)quinazolin-4-yl)oxy)phenyl)-1,2-dimet hyl-4-oxo-6-(trifluoromethoxy)-1,4-dihydroquinoline-3-carboxamide,
*N*-(3-fluoro-4-((6-methoxy-7-(3-(4-methylpiperazin-1-yl)propoxy)quinazolin-4-yl)oxy)phenyl) -1,2-dimethyl-4-oxo-6-(trifluoromethoxy)-1,4-dihydroquinoline-3-carboxamide,
*N*-(3-fluoro-4-((6-methoxy-7-(2-(pyrrolidin-1-yl)ethoxy)quinazolin-4-yl)oxy)phenyl)-1,2-dime thyl-4-oxo-6-(trifluoromethoxy)-1,4-dihydroquinoline-3-carboxamide,
*N*-(3-fluoro-4-((6-methoxy-7-(3-methoxypropoxy)quinazolin-4-yl)oxy)phenyl)-1,2-dimethyl-4 -oxo-6-(trifluoromethoxy)-1,4-dihydroquinoline-3-carboxamide,
*N*-(3-fluoro-4-((6-methoxy-7-(3-(piperidin-1-yl)propoxy)quinazolin-4-yl)oxy)phenyl)-1,2-dim ethyl-4-oxo-6-(trifluoromethoxy)-1,4-dihydroquinoline-3-carboxamide,
*N*-(4-((7-(2-(dimethylamino)ethoxy)-6-methoxyquinazolin-4-yl)oxy)-3-fluorophenyl)-1,2-dim ethyl-4-oxo-6-(trifluoromethoxy)-1,4-dihydroquinoline-3-carboxamide,
*N*-(3-fluoro-4-((7-(isopentyloxy)-6-methoxyquinazolin-4-yl)oxy)phenyl)-1,2-dimethyl-4-oxo-6 -(trifluoromethoxy)-1,4-dihydroquinoline-3-carboxamide,
*N*-(3-fluoro-4-((6-methoxy-7-propoxyquinazolin-4-yl)oxy)phenyl)-1,2-dimethyl-4-oxo-6-(trifl uoromethoxy)-1,4-dihydroquinoline-3-carboxamide,
*N*-(4-((7-ethoxy-6-methoxyquinazolin-4-yl)oxy)-3-fluorophenyl)-1,2-dimethyl-4-oxo-6-(triflu oromethoxy)-1,4-dihydroquinoline-3-carboxamide,
*N*-(3-fluoro-4-((7-methoxy-6-(3-morpholinopropoxy)quinazolin-4-yl)oxy)phenyl)-1,2-dimethy l-4-oxo-6-(trifluoromethoxy)-1,4-dihydroquinoline-3-carboxamide,
and
*N*-(3-fluoro-4-((6-methoxy-7-(3-morpholinopropoxy)quinolin-4-yl)oxy)phenyl)-1,2-dimethyl-4-oxo -6-(trifluoromethoxy)-1,4-dihydroquinoline-3-carboxamide.

The present disclosure also provides uses of the above-mentioned quinoline or quinazoline compounds.

The specific technical solutions are as follows:

The above-mentioned quinoline or quinazoline compounds or pharmaceutically acceptable salts thereof, stereoisomers thereof, or deuterated analogs thereof for use in inhibiting the action of AXL kinase and/or Flt3 kinase.

The above-mentioned quinoline or quinazoline compounds or pharmaceutically acceptable salts thereof, stereoisomers thereof, or deuterated analogs thereof for use in preventing or treating tumors.

In some embodiments, the tumor is hematological tumor, gastrointestinal stromal tumor, histiocytic lymphoma, non-small cell lung cancer, small cell lung cancer, lung adenocarcinoma, lung squamous cell carcinoma, pancreatic cancer, breast cancer, prostate cancer, liver cancer, skin cancer, epithelial cell carcinoma, or nasopharyngeal carcinoma. The hematological tumor is preferably leukemia.

The present disclosure also provides a pharmaceutical composition for use in preventing or treating a tumor.

The specific technical solutions are as follows:

A pharmaceutical composition for use in preventing or treating a tumor, comprising an active ingredient and a pharmaceutically acceptable excipient, wherein the active ingredient comprises the above-mentioned quinoline or quinazoline compound or a pharmaceutically acceptable salt thereof, a stereoisomer thereof.

The quinoline or quinazoline compounds or pharmaceutically acceptable salts thereof, stereoisomers thereof, or pharmaceutical compositions thereof of the present disclosure may be effective in inhibiting the action of AXL protein kinase and can inhibit proliferation, migration, and invasion of various tumor cells. And based on a large number of creative experimental studies, the inventors have unexpectedly found that the introduction of trifluoromethoxy at the 6-position of 1,4-dihydroquinoline of the quinoline or quinazoline compounds of the present disclosure may greatly improve in vivo metabolic stabilities of such compounds, thus allowing the compounds have higher anti-tumor activities in vivo, while having the advantages of less toxic and side effects, and can be used to prepare drugs for preventing or treating

hyperproliferative diseases such as tumors in humans and other mammals.

## BRIEF DESCRIPTION OF THE DRAWINGS

Figure 1 shows spectra of results of the detection of compound TL134-related metabolites in hepatocytes by UPLC/Q-TOF MS method; wherein A is inactivated hepatocyte, B is human hepatocyte, and C is monkey hepatocyte.
Figure 2 shows spectra of results of the detection of compound TL134-related metabolites in hepatocytes by UPLC/Q-TOF MS method; wherein D is canine hepatocyte, E is rat hepatocyte, and F is mouse hepatocyte.
Figure 3 shows spectra of results of the detection of TL134-related metabolites in hepatocytes by UPLC-UV method (254 nm); wherein A is inactivated hepatocyte, B is human hepatocyte, and C is monkey hepatocyte.
Figure 4 shows spectra of results of the detection of TL134-related metabolites in hepatocytes by UPLC-UV method (254 nm); wherein D is canine hepatocyte, E is rat hepatocyte, and F is mouse hepatocyte.

## DETAILED DESCRIPTION OF THE EMBODIMENTS

The present disclosure will be described in further detail below with reference to the examples and drawings, but the embodiment of the present disclosure is not limited thereto.

In the compounds mentioned in the present disclosure, when any variables (for example, $R_1$, R, etc.) appear more than once in any component, the definition of each occurrence is independent of the definition of each other occurrence. Also, combinations of substituents and variables are allowed as long as such combinations stabilize the compound. The line drawn entering the ring system from a substituent represents that the indicated bond may be connected to any ring atoms that can be substituted. If the ring system is polycyclic, it means that such bond is only connected to any appropriate carbon atoms of adjacent rings. It is to be understood that those of ordinary skill in the art may select substituents and substituted forms of the compounds of the present disclosure to provide chemically stable compounds that can be easily synthesized from readily available raw materials by techniques in the art and the methods set forth below. If the substituent itself is substituted with more than one group, it should be understood that these groups may be on the same carbon atom or on different carbon atoms as long as the structure is stable.

The term "alkyl" as used herein is meant to include both branched and straight chain saturated aliphatic hydrocarbon groups having a specified number of carbon atoms. For example, the definition of "$C_1$-$C_5$" in "$C_1$-$C_5$ alkyl" includes groups having 1, 2, 3, 4 or 5 carbon atoms arranged in a straight or branched chain. For example, "$C_1$-$C_5$ alkyl" specifically includes methyl, ethyl, n-propyl, isopropyl, n-butyl, tert-butyl, isobutyl, and pentyl. The term "cycloalkyl" refers to a monocyclic saturated aliphatic hydrocarbon group having a specified number of carbon atoms. For example, "cycloalkyl" includes cyclopropyl, methyl-cyclopropyl, cyclobutyl, cyclopentyl, cyclohexyl and the like.

The term "heterocycle" or "heterocyclyl / heterocyclic group" as used herein refers to a 5- to 6-membered aromatic or non-aromatic heterocyclic ring containing 1 to 4 heteroatoms selected from O, N and S, and may include bicyclic groups. The term "heterocyclyl" therefore includes the heteroaryl groups mentioned above, as well as dihydrogenated and tetrahydrogenated analogs thereof. Further examples of "heterocyclyl" include, but are not limited to: imidazolyl, thiazolyl, isoxazolyl, oxadiazolyl, oxazolyl, oxetanyl, pyranyl, pyrazinyl, pyrazolyl, pyridazinyl, pyridyl, pyrimidinyl, pyrrolyl, quinoxalinyl, tetrazolyl, thiadiazolyl, thiazolyl, thienyl, and azolyl. The connection of heterocyclic substituents may be achieved through carbon atoms or through heteroatoms.

As understood by those skilled in the art, "halo" or "halogen" as used herein is meant to include chlorine, fluorine, bromine, and iodine.

Alkyl, cycloalkyl, aryl, heteroaryl and heterocyclyl substituents may be unsubstituted or substituted, unless otherwise defined. For example, ($C_1$~$C_6$) alkyl may be substituted with one, two or three substituents selected from a group consisting of OH, halogen, nitryl, cyano group, alkoxyl, dialkylamino group and heterocyclic group, such as morpholinyl, piperidinyl and the like.

The present disclosure includes the free form of the compound of formula I as well as the pharmaceutically acceptable salts and stereoisomers thereof. Some specific exemplary compounds herein are protonated salts of amine compounds. The term "free form" refers to amine compounds in non-salt form. The included pharmaceutically acceptable salts include not only the exemplary salts of the specific compounds described herein, but also the typical pharmaceutically acceptable salts of all compounds of formula I in free form. The free form of the specific salt of the compound may be separated using techniques known in the art. For example, the free form may be regenerated by treating the salt with an appropriate basic dilute aqueous solution, such as NaOH dilute aqueous solution, potassium carbonate dilute aqueous solution, dilute ammonia liquor, and sodium bicarbonate dilute aqueous solution. The free form is somewhat different from its respective salt form in certain physical properties, such as solubility in polar solvents, but for the purposes of the invention, such acid salts and base salts are comparable to their respective free forms in other pharmaceutical aspects.

The pharmaceutically acceptable salts of the present disclosure may be synthesized from the compounds of the present disclosure containing a basic or acidic moiety by conventional chemical methods. Generally, salts of alkaline compounds are prepared by ion exchange chromatography or by the reaction of a free base and a stoichiometric amount of or an excess of inorganic or organic acids of a desired salt form in an appropriate solvent or a combination of multiple solvents. Similarly, salts of acidic compounds are formed by the reaction with appropriate inorganic or organic bases.

Therefore, the pharmaceutically acceptable salts of the compounds of the present disclosure include the conventional non-toxic salts of the compounds of the present disclosure formed by the reaction of alkali compounds of the present disclosure with inorganic or organic acids. For example, conventional non-toxic salts include salts derived from inorganic acids such as hydrochloric acid, hydrobromic acid, sulfuric acid, sulfamic acid, phosphoric acid, nitric acid, etc., as well as salts prepared from organic acids such as acetic acid, propionic acid, succinic acid, glycollic acid, stearic acid, lactic acid, malic acid, tartaric acid, citric acid, ascorbic acid, pamoic acid, maleic acid, hydroxymaleic acid, phenylacetic acid, glutamic acid, benzoic acid, salicylic acid, p-aminobenzenesulfonic acid, 2-acetoxybenzoic acid, fumaric acid, toluenesulfonic acid, methanesulfonic acid, ethanedisulfonic acid, oxalic acid, isethionic acid, trifluoroacetic acid and the like.

If the compound of the present disclosure is acidic, an appropriate "pharmaceutically acceptable salt" refers to a salt prepared by pharmaceutically acceptable non-toxic bases including inorganic bases and organic bases. Salts derived from inorganic bases include aluminum salts, ammonium salts, calcium salts, copper salts, iron salts, ferrous salts, lithium salts, magnesium salts, manganese salts, manganous salts, potassium salts, sodium salts, zinc salts, and the like. Ammonium salts, calcium salts, magnesium salts, potassium salts and sodium salts are particularly preferred. As for the salts derived from pharmaceutically acceptable organic non-toxic bases, said bases include salts of primary amines, secondary amines and tertiary amines, substituted amines include naturally occurring substituted amines, cyclic amines and basic ion exchange resins, such as arginine, glycine betaine, caffeine, choline, N,N'-dibenzylethylenediamine, diethylamine, 2-diethylaminoethanol, 2-dimethylaminoethanol, aminoethanol, ethanolamine, ethanediamine, N-ethylmorpholine, N-ethylpiperidine, glucosamine, aminoglucose, histidine, hydroxycobalamin, isopropylamine, lysine, methylglucosamine, morpholine, piperazine, piperidine, polyamine resin, procaine, purine, theobromine, triethylamine, trimethylamine, tripropylamine, tromethamine, etc.

The preparations of the above-mentioned pharmaceutically acceptable salts and other typical pharmaceutically acceptable salts are described in more detail in "Berg et al., Pharmaceutical Salts, J. Pharm. Sci. 1977: 66: 1-19".

Since the deprotonated acidic moiety of the compound such as carboxyl group may be anionic under physiological conditions, and this charge can then be counterbalanced by a protonated or alkylated basic moiety with a cation inside, such as a tetravalent nitrogen atom, and therefore it should be noted that the compounds of the present disclosure are potential inner salts or zwitterions.

In addition to the standard methods known in the literatures or exemplified in the experimental procedures, the compounds of the present disclosure may be prepared using the reactions shown in the following schemes. Therefore, the following illustrative schemes are for the purpose of illustration and are not limited to the compounds listed or any specific substituents. The number of substituents shown in the schemes does not necessarily accord with the number used in the claims, and it is shown that a mono-substituent is attached to a compound that allows multiple substituents under the definition of the formula (I) above for clarity.

Schemes **of** Synthesis

As shown in Scheme A, a compound of the formula (I) may be synthesized from 7-benzyloxy-4-chloro-6-methoxyquinazoline as a starting material through a 4-step reaction.

Scheme A:

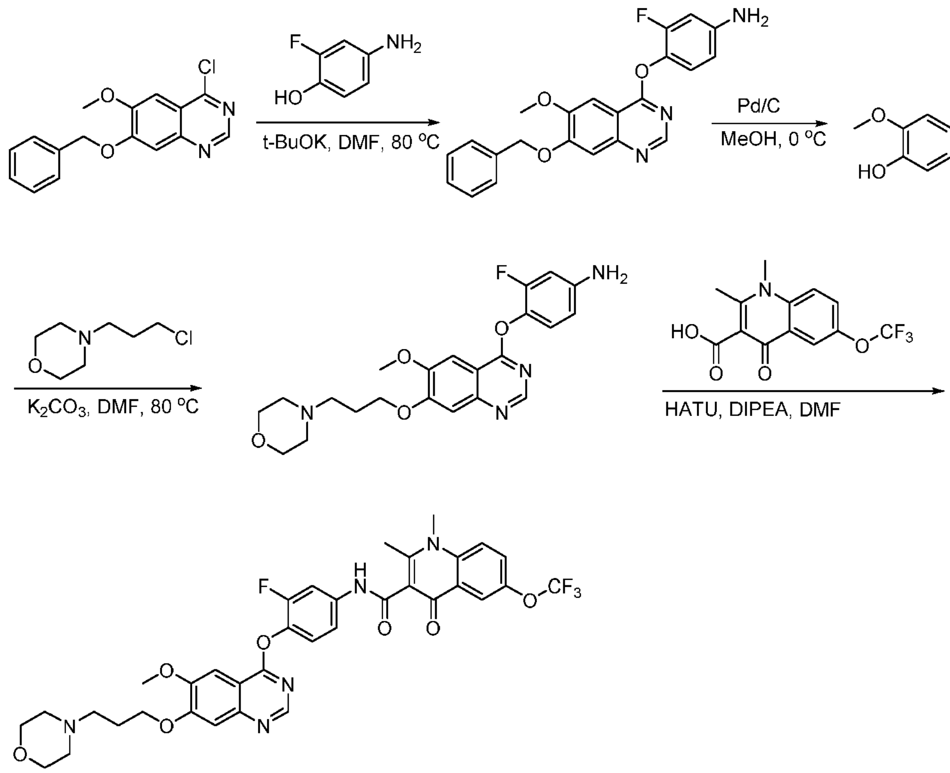


The compounds of the formula (I) provided by the present disclosure or pharmaceutically acceptable salts thereof or stereoisomers thereof may be used to treat hyperproliferative diseases or symptoms in humans or other mammals, such as tumors. Especially used in the preparation of drugs for the treatment or control of hyperproliferative diseases, such as gastrointestinal stromal tumor, histiocytic lymphoma, non-small cell lung cancer, small cell lung cancer, lung adenocarcinoma, lung squamous cell carcinoma, pancreatic cancer, breast cancer, prostate cancer, liver cancer, skin cancer, epithelial cell carcinoma, prostate cancer, nasopharyngeal carcinoma, leukemia, and the like.

The compounds designed by the present disclosure or pharmaceutically acceptable salts thereof or stereoisomers thereof may be used in combination with medicines currently in use or in the development stage to increase their clinical effects, such medicines like estrogen receptor modulators, androgen receptor modulators, retina-like receptor modulators, cytotoxins/cytostatics, antiproliferative agents, protein transferase inhibitors, HMG-CoA reductase inhibitors, HIV protein kinase inhibitors, reverse transcriptase inhibitors, angiogenesis inhibitors, cell proliferation and survival signal inhibitors, drugs that interfere with cell cycle checkpoints and apoptosis inducer, cytotoxic drugs, tyrosine protein inhibitors, EGFR inhibitors, VEGFR inhibitors, serine/threonine protein inhibitors, Bcr-Abl inhibitors, c-Kit inhibitors, Met inhibitors, Raf inhibitors, MEK inhibitors, MMP inhibitors, topoisomerase inhibitors, histidine deacetylase inhibitors, proteasome inhibitors, CDK inhibitors, Bcl-2 family protein inhibitors, MDM2 family protein inhibitors, IAP family protein inhibitors, STAT family protein inhibitors, PI3K inhibitors, AKT inhibitors, integrin blockers, interferon-a, interleukin-12, COX-2 inhibitors, p53 activators, VEGF antibodies, EGF antibodies, etc.

The compounds of the formula (I) or pharmaceutically acceptable salts thereof or stereoisomers thereof or pharmaceutical compositions thereof according to the present disclosure may be for use in prevention or treatment of the following diseases and other diseases not listed below:

(1) Breast cancers in humans or other mammals, including but not limited to invasive ductal carcinoma, invasive lobular carcinoma, ductal carcinoma in situ, and lobular carcinoma in situ.

(2) Respiratory tract cancers in humans or other mammals, including but not limited to small cell lung cancer, non-small cell lung cancer and bronchial adenoma and pleuropulmonary blastoma.

(3) Brain cancers in humans or other mammals, including but not limited to brainstem and subocular gliomas, cerebellar and cerebral astrocytomas, ependymoma, and neuroectodermal and pineal tumors.

(4) Tumors in male and female reproductive organs of humans or other mammals, tumors of male reproductive organs including but not limited to prostate and testicular cancers; tumors of female reproductive organs including but not limited to endometrial cancer, cervical cancer, ovarian cancer, vaginal cancer and vulvar cancer, and intrauterine tumor.

(5) Tumors in the digestive tracts of humans or other mammals, including but not limited to anal cancer, colon cancer, colorectal cancer, esophageal cancer, gastric cancer, pancreatic cancer, rectal cancer, small intestine cancer, or salivary gland cancer.
(6) Tumors in the urethras of humans or other mammals, including but not limited to bladder cancer, penile cancer, kidney cancer, renal pelvis cancer, ureteral cancer or urethral cancer.
(7) Eye cancers in humans or other mammals, including but not limited to intraocular melanoma and retinocytoma.
(8) Liver cancers in humans or other mammals, including but not limited to hepatocellular carcinoma (stem cell carcinoma with or without fiberboard changes), cholangiocarcinoma (intrahepatic cholangiocarcinoma), and mixed hepatocellular cholangiocarcinoma.
(9) Skin cancers in humans or other mammals, including but not limited to squamous cell carcinoma, Kaposi's sarcoma, malignant melanoma, Merck's cells skin cancer, and non-melanoma cell carcinoma.
(10) Head and neck cancers in humans or other mammals, including but not limited to the cancers of larynx, hypopharynx, nasopharynx, oropharynx, and lip and oral cancers.
(11) Lymphomas in human or other mammals, including but not limited to AIDS-related lymphoma, non-Hodgkin's lymphoma, cutaneous T-cell lymphoma, Hodgkin's disease, and central nervous system lymphoma.
(12) Sarcomas in humans or other mammals, including but not limited to soft tissue sarcoma, osteosarcoma, malignant fibrous histiocytoma, lymphatic sarcoma and rhabdomyosarcoma.
(13) Leukemias in humans or other mammals, including but not limited to acute myeloid leukemia, acute lymphocytic leukemia, chronic lymphocytic leukemia, chronic myelogenous leukemia, and hairy cell leukemia.

**Mode of Administration and Dosage Range**

According to standard pharmaceutical techniques, the compounds of the present disclosure may be administrated alone or in combination with pharmaceutically acceptable receptors, excipients or diluents in pharmaceutical compositions, to a mammal, preferably a human. The compounds may be administered via oral or subcutaneous, intramuscular, intraperitoneal, intravenous, rectal and topical, eyes, lungs, nasal cavities, and parenteral.
In one embodiment, the dosage range is from 0.1 to 500 mg/day/kg of body weight orally when the compounds of the formula (I) are used to prepare drugs for the treatment or control of the patients with cancer and the like. The appropriate mode of administration is single-dose daily administration, or two-, three- or four-times daily administration, or administration using sustained-release techniques. For a variety of large mammals, the preferred dosage range thereof is from 0.1 to 1500 mg/day/kg of body weight, preferably from 0.5 to 100 mg/day/kg of body weight. For patients with an average weight of 70 kg, the daily dosage thereof is from 1 to 500 mg. For some particularly high active compounds, the daily dosage for adult patients may be as low as 0.1 mg/day.

**Combination Administration**

The compounds of the formula (I) may be used in combination with known drugs for treating or ameliorating similar symptoms. In the combination administration, the administration method and dosage of the known drugs have been remained the same, while a compound of the formula (I) is taken contemporaneously or sequentially. When a compound of the formula (I) is used contemporaneously with one or more other drugs, a pharmaceutical composition containing one or more other known drugs and the compound of the formula (I) is preferably used. The combination drug therapy also comprises taking the compound of the formula (I) with one or more other known drugs in overlapping time periods. When the compound of the formula (I) is used in combination with one or more other drugs, the compound of the formula (I) and the other known drugs may be used in lower dosage than when they are used alone.
Drugs or active ingredients that can be used in combination with the compounds of the formula (I) include but are not limited to:
estrogen receptor modulators, androgen receptor modulators, retina-like receptor modulators, cytotoxins/cytostatics, antiproliferative agents, protein transferase inhibitors, HMG-CoA reductase inhibitors, HIV protein kinase inhibitors, reverse transcriptase inhibitors, angiogenesis inhibitors, cell proliferation and survival signal inhibitors, drugs that interfere with cell cycle checkpoints and apoptosis inducer, cytotoxic drugs, tyrosine protein inhibitors, EGFR inhibitors, VEGFR inhibitors, serine/threonine protein inhibitors, Bcr-Abl inhibitors, c-Kit inhibitors, Met inhibitors, Raf inhibitors, MEK inhibitors, MMP inhibitors, topoisomerase inhibitors, histidine deacetylase inhibitors, proteasome inhibitors, CDK inhibitors, Bcl-2 family protein inhibitors, MDM2 family protein inhibitors, IAP family protein inhibitors, STAT family protein inhibitors, PI3K inhibitors, AKT inhibitors, integrin blockers, interferon-a, interleukin-12, COX-2 inhibitors, p53, p53 activators, VEGF antibodies, EGF antibodies, and the like.
In one embodiment, drugs or active ingredients that can be used in combination with the compounds of the formula (I) include, but are not limited to: aldesleukin, alendronic acid, interferon, alitretinoin, allopurinol, sodium allopurinol, palonosetron hydrochloride, altretamine, aminoglutethimide, amifostine, amrubicin, amsacrine, anastrozole, dolasetron, aranesp, arglabin, arsenic trioxide, aromasin , 5-azacytidine, azathioprine, bacillus calmette-guerin (BCG) or tice BCG, bestatin, betamethasone acetate, betamethasone sodium phosphate preparation, bexarotene, bleomycin sulfate, bromouridine, bortezomib, busulfan, calcitonin, alemtuzumab injection, capecitabine, carboplatin, casodex, cefesone, celmoleukin, daunorubicin, chlorambucil, cis-platinum, cladribine, clodronic acid, cyclophosphamide, cytarabine, dacarbazine, actinomycin D, daunorubicin liposomes, dexamethasone, dexamethasone phosphate, estradiol valerate, denileukin diftitox 2, depo-medrol, deslorellin, dexrazoxane, stilbestrol, diflucan, docetaxel, doxifluridine, adriamycin, dronabinol, Ho-166-chitosan complex, eligard, rasburicase, epirubicin hydrochloride, aprepitant, epirubicin, epoetin alfa, erythropoietin, eptaplatin, levamisole tablets, estradiol preparations, 17-β-estradiol, estramustine sodium phosphate, ethinylestradiol, amifostine, hydroxyphosphoric acid, etopophos, etoposide, fadrozole, tamoxifen preparations, filgrastim, finasteride, filgrastim, floxuridine, fluconazole, fludarabine, 5-fluoro-2-deoxyuridine monophosphate, 5-fluorouracil, fluoxymesterone, flutamide, formestan, 1-β-D-arabinofuranosylcytosine-5'-stearoyl phosphate, fotemustine, fulvestrant, gamma globulin, gemcitabine, gemituzumab, imatinib mesylate, carmustine glutinous rice paper capsules, goserelin, granitelon hydrochloride, histralin, hycamtin, hydrocortisone, erythro-hydroxynonyladenine, hydroxyurea, ibritumomab tiuxetan, idarubicin, ifosfamide, interferon α, interferon-α2, interferon α-2A, interferon α-2B, interferon α-nl, interferon α-n3, interferonβ, interferon γ-la, interleukin-2, intron A, iressa, irinotecan, kytril, lentinan sulfate, letrozole, formyltetrahydrofolate, leuprorelin, leuprorelin acetate, levamisole, calcium levofolinate, levothyroxine sodium, levothyroxine sodium preparations, lomustine, lonidamine, dronabinol, nitrogen mustard, methylcobalamin, medroprogesterone acetate, megestrol acetate, melphalan, esterified estrogen, 6-mercaptopurine, mesna, amethopterin, methyl aminolevulinate, miltefosine, minocycline, mitomycin C, mitotane, mitoxantrone, trilostane, adriamycin citrate liposomes, nedaplatin, pegylated filgrastim, oprelvekin, neupogen, nilutamide, tamoxifen, NSC-631570, recombinant human interleukin 1-β, octreotide, ondansetron hydrochloride, dehydrohydrocortisone oral solutions, oxaliplatin, paclitaxel, prednisone sodium phosphate preparations, pegaspargase, pegasys, pentostatin, picibanil, pilocarpine hydrochloride, pirarubicin, plicamycin, porfimer sodium, prednimustine, prednisolone steaglate, prednisone, premarin, procarbazine, recombinant human erythropoietin, raltitrexed, rebif, etidronate-rhenium-186, mabthera, redoxon-A, romurtide, pilocarpine hydrochloride tablets, octreotide, sargramostim, semustine, sizofiran, sobuzoxane, methylprednisolone sodium, paphos acid, stem cell therapy, streptozocin, strontium chloride-89, levothyroxine sodium, tamoxifen, tamsulosin, tasunaming, tastolactone, taxotere, teceleukin, temozolomide, teniposide, testosterone propionate, methyltestosterone, thioguanine, thiotepa, thyroid stimulating hormone, tiludronic acid, topotecan, toremifene, tositumomab, trastuzumab, treosulfan, tretinoin, methotrexate tablets, trimethylmelamine, trimetrexate, triptorelin acetate, triptorelinpamoate, UFT, uridine, valrubicin, vesnarinone, vinblastine, vincristine, vindesine, vinorelbine, virulizin, dexrazoxane, zinostatin stimalamer, zofran, paclitaxel protein stabilizer, acolbifene, interferon r-lb, affinitak, aminopterin, arzoxifene, asoprisnil, atamestane, atrasentan, BAY 43-9006, avastin, CCI-779, CDC-501, celebrex, cetuximab, crisnatol, cyproterone acetate, decitabine, DN-101, adriamycin-MTC, dSLIM, dutasteride, edotecarin, eflunithine, exatecan, fenretinide, histamine dihydrochloride, histrelin hydrogel implant, holmium-166 DOTMP, ibandronic acid, interferon γ, intron-PEG, ixabepilone, keyhole limpet haemocyanine, L-651582, lanreotide, lasofoxifene, libra, lonafamib, miproxifene, minocolate, MS-209, lipidosome MTP-PE, MX-6, nafarelin, nemorubicin, neovastat, nolatrexed, oblimersen, onco-TCS, osidem, paclitaxel polyglutamate, sodium pamidronate, PN-401, QS-21, quazepam, R-1549, raloxifene, onconase, 13-cis-retinoic acid, satraplatin, seocalcitol, T-138067, tarceva, docosahexaenoic acid paclitaxel, thymosin αl, galazolin, tipifarnib, tirapazamine, TLK-286, toremifene, trans-MID-lo7R, valspodar, vapreotide, vatalanib, verteporfin, vinflunine, Z-100 and zoledronic acid or a combination thereof.

The following are specific examples, and the raw material reagents used in the following examples are all commercially available.

**Example 1:** Preparation of *N*-(3-fluoro-4-((6-methoxy-7-(3-morpholinopropoxy)quinazolin-4-yl)oxy)phenyl)-1,2-dim ethyl-4-oxo-6-(trifluoromethoxy)-1,4-dihydroquinoline-3-carboxamide (named as **TL134)**

a

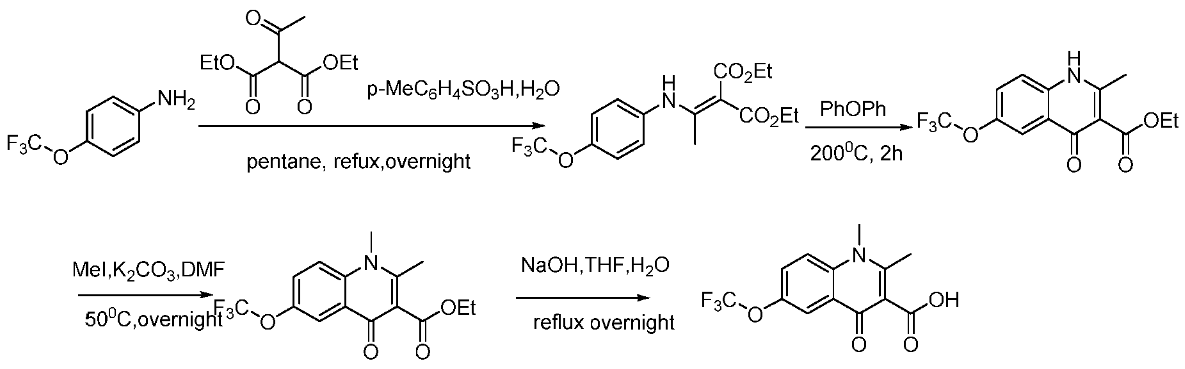


Step a1: Preparation of diethyl 2-(1-((4-(trifluoromethoxy)phenyl)amino)ethylidene)malonate

P-trifluoromethoxyaniline (2.42 g, 20 mmol) and diethyl acetylmalonate (2.02 g, 10 mmol) were dissolved in 50 mL of n-pentane, then a catalytic amount of p-toluenesulfonic acid (20 mg) was added, and the reaction was refluxed overnight. The reaction was cooled to room temperature, and a small amount of saturated $NaHCO_3$ was added, then the mixture was extracted twice with EA. The organic phases were combined, washed once with saturated brine, and dried over anhydrous $Na_2SO_4$, filtered and dried by rotary evaporation, and then subjected to column chromatography to give 2.68 g (87.8%) of a solid.

Step a2: Preparation of ethyl 2-methyl-4-oxo-6-(trifluoromethoxy)-1,4-dihydroquinoline-3-carboxylate

Diethyl 2-(1-((4-(trifluoromethoxy)phenyl)amino)ethylidene)malonate (2.5 g, 8.2 mmol) was dissolved in 25 mL of diphenyl ether, heated to 200 °C, and reacted with stirring for 2 hours. The reaction was cooled to room temperature, and a solid precipitated, then the mixture was filtered, washed with PE, and then suction-dried to give 2 g (94.3%) of a white solid.

Step a3: Preparation of ethyl 1,2-dimethyl-4-oxo-6-(trifluoromethoxy)-1,4-dihydroquinoline-3-carboxylate

Ethyl 2-methyl-4-oxo-6-(trifluoromethoxy)-1,4-dihydroquinoline-3-carboxylate (2 g, 7.7 mmol) and $K_2CO_3$ (3.18 g, 23.1 mmol) were dissolved in 50 mL of DMF, then MeI (0.72 mL, 11.55 mmol) was added with stirring, and the mixture was reacted at 50 °C overnight. The reaction was cooled to room temperature, quenched with water, then a solid precipitated. The mixture was washed with water multiple times, and the solid was extracted with DCM multiple times. The organic phases were combined and dried by rotary evaporation, and then subjected to column chromatography to give 1.52 g (72.4%) of a white solid.

Step a4: Preparation of 1,2-dimethyl-4-oxo-6-(trifluoromethoxy)-1,4-dihydroquinoline-3-carboxylic acid

Ethyl 1,2-dimethyl-4-oxo-6-(trifluoromethoxy)-1,4-dihydroquinoline-3-carboxylate (1.5 g, 5.5 mmol) and NaOH (880 mg, 22 mmol) were dissolved in a mixed solvent of 30 mL of ethanol and 15 mL of water, then the mixture was reacted overnight. The reaction was cooled to room temperature, then most of the organic solvent was dried by rotary evaporation, added water, adjusted pH to 7-8 by dilute HCl in an ice bath, then a solid precipitated, the mixture was filtered and then suction-dried to give 1.25 g (93.3%) of a white solid.

b

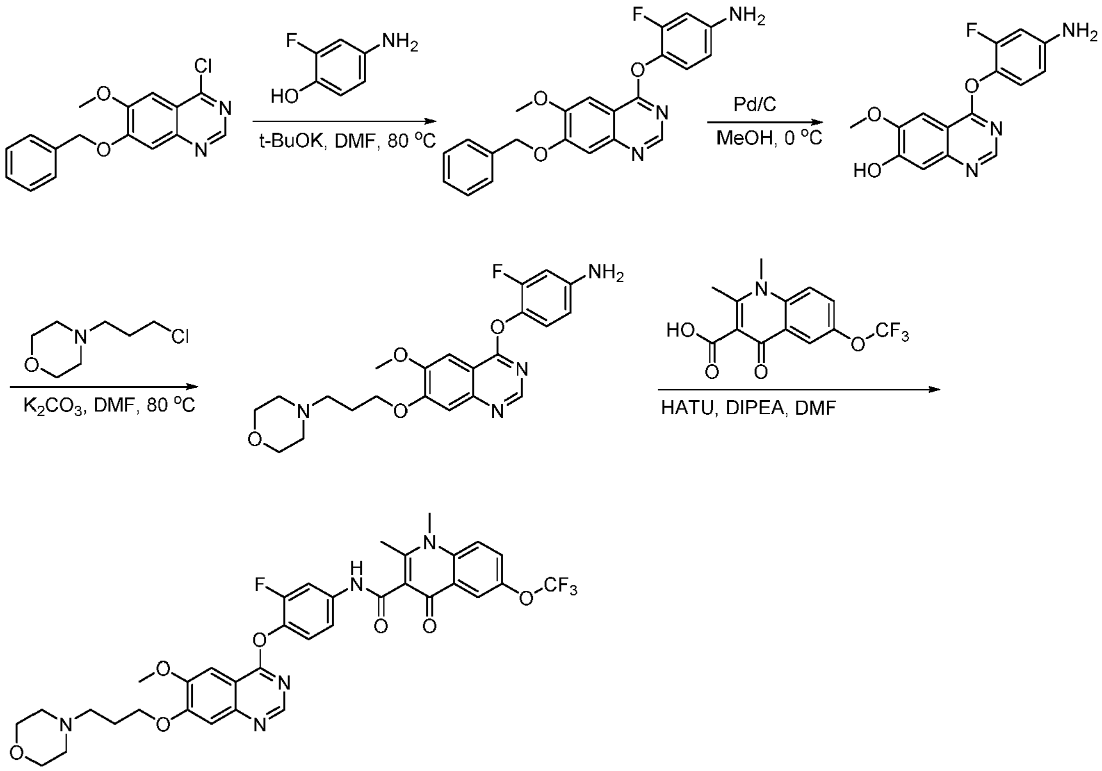


Step b1: Preparation of 4-((7-(benzyloxy)-6-methoxyquinazolin-4-yl)oxy)-3-fluoroaniline

To a reaction flask were added 7- benzyloxy-4-chloro-6-methoxyquinazoline (4.5 g, 15 mmol), 4-amino-2-fluorophenol (2.3 g, 18 mmol), potassium tert-butoxide (2.4 g, 21 mmol) and DMF (250 mL), and the mixture was heated to 80 °C for reaction for 2 hours. After the reaction was stopped and the solvent was removed under reduced pressure, the mixture was subjected to dry column chromatography to give 3.6 g (62%) of 4-((7-(benzyloxy) -6-methoxyquinazolin-4-yl)oxy)-3-fluoroaniline. $^1$H NMR (400 MHz, $d_6$-DMSO) $\delta$ 8.53 (s, 1 H), 7.55 (s, 1 H), 7.52 (m, 2 H), 7.49 (s, 1 H), 7.44 (t, $J$ = 7.2 Hz, 2 H), 7.37 (t, $J$ = 7.2 Hz, 1 H), 7.04 (t, $J$ = 8.8 Hz, 1 H), 6.50 (dd, $J$ = 2.4, 13.2 Hz, 1 H), 6.42 (dd, $J$ = 2.4, 8.8 Hz, 1 H), 5.39 (s, 2 H), 5.35 (s, 2 H), 3.97 (s, 3 H). MS (ESI), m/z: 391 $[M+H]^+$.

Step b2: Preparation of 4-(4-amino-2-fluorophenoxy)-6-methoxyquinazolin-7-ol

4-((7-(benzyloxy)-6-methoxyquinazolin-4-yl)oxy)-3-fluoroaniline (5.2 g, 13.3 mmol), Pd/C (0.4 g) and methanol (250 mL) were reacted at 0 °C under a hydrogen atmosphere overnight, then Pd/C was removed by filtration, and the filtrate was concentrated and subjected to a column to give 2.4 g (60%) of 4-(4-amino-2-fluorophenoxy)-6-methoxyquinazolin-7-ol. $^1$H NMR (400 MHz, $d_6$-DMSO) $\delta$ 10.72 (s, 1 H), 8.45 (s, 1 H), 7.52 (s, 1 H), 7.22 (d, $J$ = 3.2 Hz, 1 H), 7.02 (t, $J$ = 8.8 Hz, 1 H), 6.49 (dd, $J$ = 2.4, 12.8 Hz, 1 H), 6.41 (dd, $J$ = 2.0, 8.8 Hz, 1 H), 5.37 (s, 2 H), 3.97 (s, 3 H). MS (ESI), m/z: 301 $[M+H]^+$.

Step b3: Preparation of 3-fluoro-4-((6-methoxy-7-(3-morpholinopropoxy)quinazolin-4-yl)oxy)aniline

4-(4-amino-2-fluorophenoxy)-6-methoxyquinazolin-7-ol (400 mg, 1.3 mmol), 4-(3-chloropropyl)morpholine (3-5a) (640 mg, 3.9 mmol) and potassium carbonate (540 mg, 3.9 mmol) were added to DMF (50 mL), the mixture was heated to 80 °C and reacted for two hours, then extracted three times with ethyl acetate. The organic phases were combined, washed with saturated brine, and dried by rotary evaporation, and then subjected to a column to give 380 mg (67%) of 3-fluoro-4-((6-methoxy-7-(3-morpholinopropoxy)quinazolin-4-yl)oxy)aniline. $^1$H NMR (400 MHz, $CDCl_3$) $\delta$ 8.60 (s, 1 H), 7.53 (s, 1 H), 7.31 (s, 1 H), 7.05 (t, $J$ = 8.8 Hz, 1 H), 6.49 (dd, $J$ = 2.4, 12.0 Hz, 1H), 6.41 (dd, $J$ = 2.4, 8.8 Hz, 1H), 4.26 (t, $J$ = 6.4 Hz, 2 H), 4.02 (s, 3 H), 3.71 (t, $J$ = 4.4 Hz, 4 H), 2.56 (t, $J$ = 7.2 Hz, 2 H), 2.47 (s, 4 H), 2.11 (m, 2 H). MS (ESI), m/z: 428 $[M+H]^+$.

Step b4: Preparation of *N*-(3-fluoro-4-((6-methoxy-7-(3-morpholinopropoxy)quinazolin-4-yl)oxy)phenyl)-1,2-dimethyl-4-o xo-6-(trifluoromethoxy)-1,4-dihydroquinoline-3-carboxamide (named as **TL134)**

3-fluoro-4-((6-methoxy-7-(3-morpholinopropoxy)quinazolin-4-yl)oxy)aniline (450 mg, 1 mmol), 1,2-dimethyl-4-oxo-6-(trifluoromethoxy)-1,4-dihydroquinoline-3-carboxylic acid (277 mg, 1.2 mmol), HATU (570 mg, 1.5 mmol) and DIEA (0.5 mL, 3 mmol) were dissolved in 30 mL of DMF, then the mixture was stirred overnight at room temperature. Ice water was added to the reaction solution, then a solid precipitated. The mixture was filtered, and the solid was extracted twice with dichloromethane. The organic phases were combined and washed once with saturated brine, dried over anhydrous $Na_2SO_4$, then filtered and dried by rotary evaporation, and then subjected to column chromatography to give 478 mg (72%) of a white solid.

Example 2: Preparation of *N*-(4-((6,7-bis(2-methoxyethoxy)quinazolin-4-yl)oxy)-3-fluorophenyl)-1,2-dimethyl-4-oxo-6-(triflu oromethoxy)-1,4-dihydroquinoline-3-carboxamide (named as **TL197)**

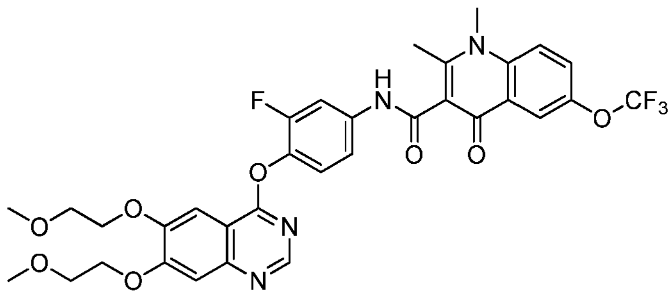

The synthesis method is referred to Example 1.

$^1H$ NMR (400 MHz, DMSO-$d_6$) δ 10.78 (s, 1H), 8.56 (s, 1H), 8.12 - 8.04 (m, 2H), 7.94 (dd, *J* = 12.8, 2.3 Hz, 1H), 7.82 (dd, *J* = 9.3, 3.0 Hz, 1H), 7.63 (s, 1H), 7.53 - 7.39 (m, 3H), 4.35 (ddd, *J* = 9.1, 4.4, 2.7 Hz, 4H), 3.88 (s, 3H), 3.77 (q, *J*= 4.9 Hz, 4H), 3.36 (s, 5H), 2.63 (s, 3H).

Example 3: Preparation of *N*-(4-((6,7-dimethoxyquinazolin-4-yl)oxy)-3-fluorophenyl)-1,2-dimethyl-4-oxo-6-(trifluoromethoxy )-1,4-dihydroquinoline-3-carboxamide (named as **TL198)**

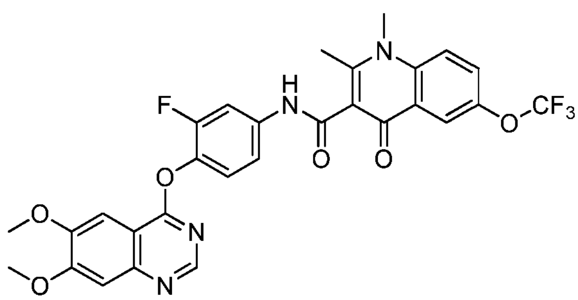

The synthesis method is referred to Example 1.

$^1H$ NMR (400 MHz, DMSO-$d_6$) δ 10.78 (s, 1H), 8.57 (s, 1H), 8.07 (d, *J* = 12.0 Hz, 2H), 7.94 (d, *J* = 12.7 Hz, 1H), 7.83 (d, *J*= 8.5 Hz, 1H), 7.59 (s, 1H), 7.54 - 7.34 (m, 3H), 4.00 (s, 6H), 3.88 (s, 3H), 2.63 (s, 3H).

Example 4: Preparation of *N*-(3-fluoro-4-((7-methoxy-6-(3-methoxypropoxy)quinazolin-4-yl)oxy)phenyl)-1,2-dimethyl-4-oxo-6-(trifluoromethoxy)-1,4-dihydroquinoline-3-carboxamide (named as **TL199)**

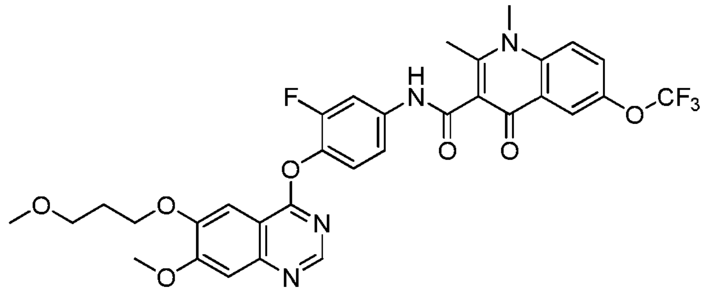

The synthesis method is referred to Example 1.

$^1$H NMR (400 MHz, Chloroform-*d*) δ 12.32 (s, 1H), 8.62 (s, 1H), 8.38 (dd, *J* = 2.7, 1.3 Hz, 1H), 7.98 (dd, *J*= 12.4, 2.5 Hz, 1H), 7.68 (d, *J*= 9.3 Hz, 1H), 7.64 - 7.57 (m, 2H), 7.47 - 7.41 (m, 1H), 7.33 (s, 1H), 7.28 (d, *J*= 8.6 Hz, 1H), 4.32 (t, *J*= 6.5 Hz, 2H), 4.05 (s, 3H), 3.95 (s, 3H), 3.62 (t, *J* = 6.1 Hz, 2H), 3.38 (s, 3H), 3.11 (s, 3H), 2.22 (p, *J* = 6.3 Hz, 2H).

Example 5: Preparation of *N*-(3-fluoro-4-((6-methoxy-7-(4-methoxybutoxy)quinazolin-4-yl)oxy)phenyl)-1,2-dimethyl-4-oxo-6 -(trifluoromethoxy)-1,4-dihydroquinoline-3-carboxamide (named as **TL204)**

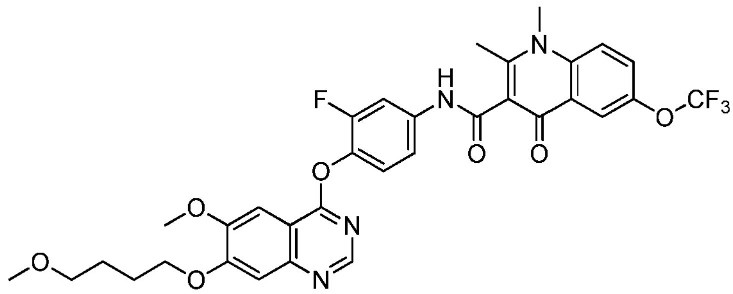

The synthesis method is referred to Example 1.

$^1$H NMR (400 MHz, Chloroform-*d*) δ 12.32 (s, 1H), 8.62 (s, 1H), 8.37 (dd, *J* = 2.9, 1.4 Hz, 1H), 7.98 (dd, *J* = 12.4, 2.4 Hz, 1H), 7.67 (d, *J* = 9.4 Hz, 1H), 7.63 - 7.58 (m, 1H), 7.56 (s, 1H), 7.43 (ddd, *J* = 8.7, 2.4, 1.2 Hz, 1H), 7.30 (d, *J* = 13.5 Hz, 2H), 4.24 (t, *J* = 6.6 Hz, 2H), 4.05 (s, 3H), 3.95 (s, 3H), 3.48 (t, *J* = 6.4 Hz, 2H), 3.36 (s, 3H), 3.11 (s, 3H), 2.10 - 1.98 (m, 2H), 1.88 - 1.76 (m, 2H).

Example 6: Preparation of *N*-(3-fluoro-4-((6-methoxy-7-(2-morpholinoethoxy)quinazolin-4-yl)oxy)phenyl)-1,2-dimethyl-4-ox o-6-(trifluoromethoxy)-1,4-dihydroquinoline-3-carboxamide (named as **TL209)**

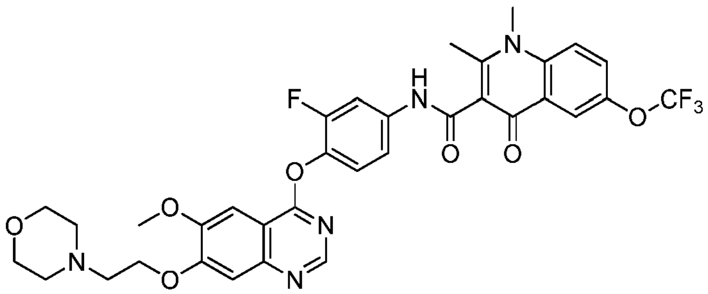

The synthesis method is referred to Example 1.

$^1$H NMR (400 MHz, Chloroform-*d*) δ 12.34 (s, 1H), 8.63 (s, 1H), 8.39 (dd, *J* = 2.9, 1.3 Hz, 1H), 7.99 (dd, *J* = 12.4, 2.4 Hz, 1H), 7.68 (d, *J* = 9.3 Hz, 1H), 7.64 - 7.55 (m, 2H), 7.44 (ddd, *J* = 8.8, 2.5, 1.3 Hz, 1H), 7.33 (s, 1H), 7.30 (d, *J*= 8.6 Hz, 1H), 4.36 (s, 2H), 4.06 (s, 3H), 3.96 (s, 3H), 3.78 (d, *J* = 5.2 Hz, 4H), 3.12 (s, 3H), 2.97 (s, 2H), 2.66 (s, 4H).

Example 7: Preparation of *N*-(3-fluoro-4-((6-methoxy-7-(3-(pyrrolidin-1-yl)propoxy)quinazolin-4-yl)oxy)phenyl)-1,2-dimethy l-4-oxo-6-(trifluoromethoxy)-1,4-dihydroquinoline-3-carboxamide (named as **TL212)**

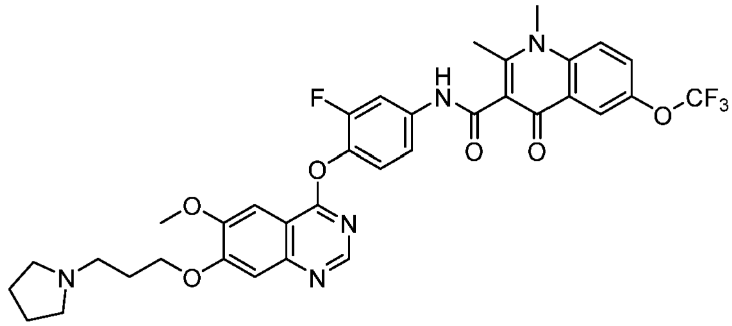

The synthesis method is referred to Example 1.

$^1$H NMR (400 MHz, Chloroform-*d*) δ 12.33 (s, 1H), 8.62 (s, 1H), 8.39 (s, 1H), 7.99 (dd, *J* = 12.3, 2.4 Hz, 1H), 7.68 (d, *J*= 9.3 Hz, 1H), 7.64 - 7.58 (m, 1H), 7.57 (s, 1H), 7.44 (d, *J*= 8.9 Hz, 1H), 7.34 (s, 1H), 7.30 (s, 1H), 4.29 (t, *J* = 6.7 Hz, 2H), 4.06 (s, 3H), 3.96 (s, 3H), 3.11 (s, 2H), 2.69 (t, *J* = 7.5 Hz, 2H), 2.56 (s, 4H), 2.24 - 2.11 (m, 2H), 2.05 (s, 1H), 1.80 (t, *J* = 4.8 Hz, 4H), 1.25 (q, *J* = 6.9, 6.4 Hz, 2H).

Example 8: Preparation of *N*-(3-fluoro-4-((6-methoxy-7-(2-(piperidin-1-yl)ethoxy)quinazolin-4-yl)oxy)phenyl)-1,2-dimethyl-4 -oxo-6-(trifluoromethoxy)-1,4-dihydroquinoline-3-carboxamide (named as **TL213)**

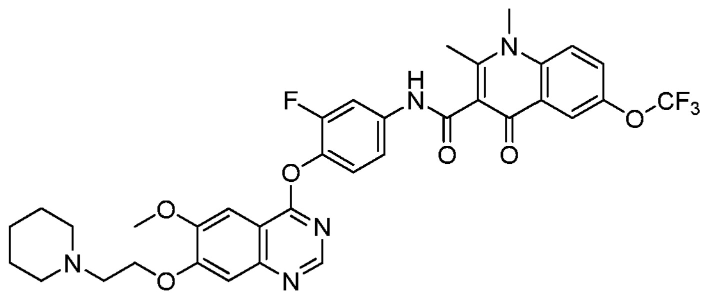

The synthesis method is referred to Example 1.

1H NMR (400 MHz, Chloroform-d) δ 12.32 (s, 1H), 8.62 (s, 1H), 8.38 (d, J = 2.7 Hz, 1H), 7.99 (dd, J = 12.3, 2.4 Hz, 1H), 7.68 (d, J = 9.3 Hz, 1H), 7.63 - 7.54 (m, 2H), 7.44 (dd, J = 8.8, 2.0 Hz, 1H), 7.33 (s, 1H), 7.29 (d, J = 8.8 Hz, 1H), 4.39 (t, J = 6.1 Hz, 2H), 4.05 (s, 3H), 3.96 (s, 3H), 3.11 (s, 3H), 3.02 (s, 2H), 2.67 (s, 4H), 1.78 - 1.62 (m, 6H).

Example 9: Preparation of *N*-(3-fluoro-4-((6-methoxy-7-(3-(4-methylpiperazin-1-yl)propoxy)quinazolin-4-yl)oxy)phenyl)-1,2-dimethyl-4-oxo-6-(trifluoromethoxy)-l,4-dihydroquinoline-3-carboxamide (named as **TL238)**

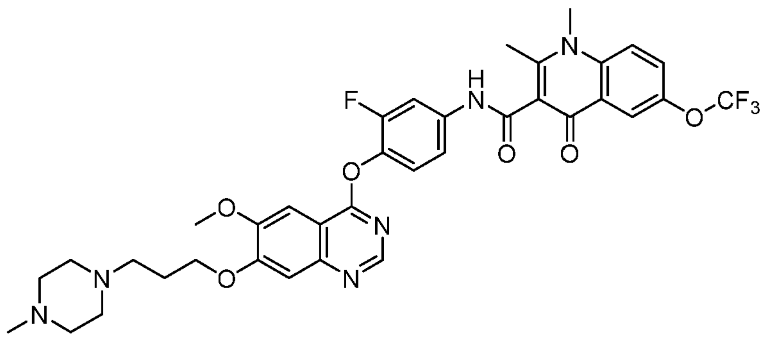

The synthesis method is referred to Example 1.

1H NMR (400 MHz, DMSO-d6) δ 10.80 (s, 1H), 8.56 (s, 1H), 8.13 - 8.03 (m, 2H), 7.94 (dd, J = 12.8, 2.4 Hz, 1H), 7.82 (dd, J = 9.3, 3.1 Hz, 1H), 7.59 (s, 1H), 7.50 (dd, J = 8.9, 2.3 Hz, 1H), 7.44 (t, J = 8.6 Hz, 1H), 7.40 (s, 1H), 4.24 (t, J = 6.4 Hz, 2H), 3.99 (s, 3H), 3.88 (s, 3H), 2.63 (s, 3H), 2.45 (t, J = 7.0 Hz, 3H), 2.33 (s, 6H), 2.15 (s, 3H), 1.98 (q, J = 6.9, 6.4 Hz, 3H), 1.55 (s, 1H).

Example 10: Preparation of *N*-(3-fluoro-4-((6-methoxy-7-(2-(pyrrolidin-1-yl)ethoxy)quinazolin-4-yl)oxy)phenyl)-1,2-dimethyl-4-oxo-6-(trifluoromethoxy)-1,4-dihydroquinoline-3-carboxamide (named as **TL231)**

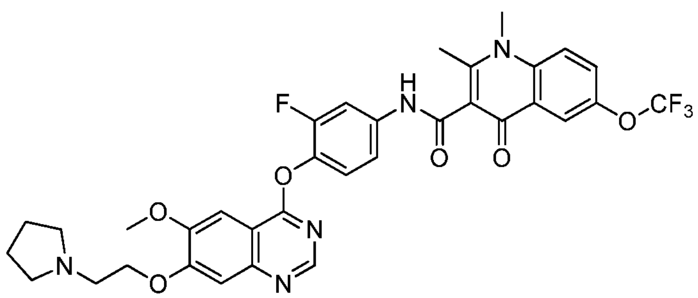

The synthesis method is referred to Example 1.

1H NMR (400 MHz, Chloroform-d) δ 12.30 (s, 1H), 8.62 (s, 1H), 8.35 (dd, J = 2.8, 1.4 Hz, 1H), 7.97 (dd, J = 12.4, 2.4 Hz, 1H), 7.66 (d, J = 9.4 Hz, 1H), 7.58 (d, J = 13.6 Hz, 2H), 7.42 (ddd, J = 8.8, 2.5, 1.2 Hz, 1H), 7.32 (s, 1H), 7.28 (d, J = 8.5 Hz, 1H), 4.35 (t, J = 6.1 Hz, 2H), 4.05 (s, 3H), 3.93 (s, 3H), 3.10 (d, J = 6.0 Hz, 2H), 3.09 (s, 3H), 2.73 (d, J = 6.0 Hz, 4H), 1.85 (p, J = 3.3 Hz, 5H).

Example 11: Preparation of *N*-(3-fluoro-4-((6-methoxy-7-(3-methoxypropoxy)quinazolin-4-yl)oxy)phenyl)-1,2-dimethyl-4-oxo-6-(trifluoromethoxy)-1,4-dihydroquinoline-3-carboxamide (named as TL226)

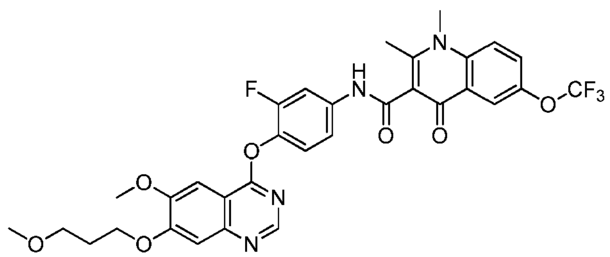

The synthesis method is referred to Example 1.

1H NMR (400 MHz, Chloroform-d) δ 12.27 (s, 1H), 8.61 (s, 1H), 8.35 - 8.29 (m, 1H), 7.97 (dd, J = 12.3, 2.4 Hz, 1H), 7.64 (d, J = 9.3 Hz, 1H), 7.58 (d, J = 3.1 Hz, 1H), 7.56 (s, 1H), 7.41 (dt, J = 8.8, 1.7 Hz, 1H), 7.34 (s, 1H), 7.30 -

7.24 (m, 1H), 4.31 (t, J = 6.5 Hz, 2H), 4.05 (s, 3H), 3.92 (s, 3H), 3.61 (t, J = 6.1 Hz, 2H), 3.38 (s, 3H), 3.07 (s, 3H), 2.21 (p, J = 6.3 Hz, 2H).

Example 12: Preparation of *N*-(3-fluoro-4-((6-methoxy-7-(3-(piperidin-1-yl)propoxy)quinazolin-4-yl)oxy)phenyl)-1,2-dimethyl -4-oxo-6-(trifluoromethoxy)-1,4-dihydroquinoline-3-carboxamide (named as **TL216)**

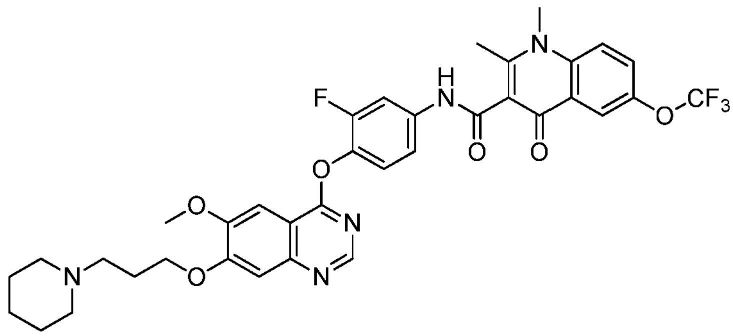

The synthesis method is referred to Example 1.

1H NMR (400 MHz, DMSO-d6) δ 10.78 (s, 1H), 8.55 (s, 1H), 8.12 - 8.04 (m, 2H), 7.94 (dd, J = 12.9, 2.3 Hz, 1H), 7.82 (dd, J = 9.3, 3.0 Hz, 1H), 7.59 (s, 1H), 7.53 - 7.37 (m, 3H), 4.24 (t, J = 6.5 Hz, 2H), 4.00 (d, J = 2.5 Hz, 3H), 3.88 (s, 3H), 3.33 (s, 3H), 2.63 (s, 3H), 2.42 (t, J = 7.1 Hz, 2H), 2.35 (s, 4H), 2.02 - 1.90 (m, 2H), 1.51 (p, J = 5.5 Hz, 4H), 1.39 (q, J = 6.7, 6.2 Hz, 2H).

Example 13: Preparation of *N*-(4-((7-(2-(dimethylamino)ethoxy)-6-methoxyquinazolin-4-yl)oxy)-3-fluorophenyl)-1,2-dimethyl-4-oxo-6-(trifluoromethoxy)-1,4-dihydroquinoline-3-carboxamide (named as **TL233)**

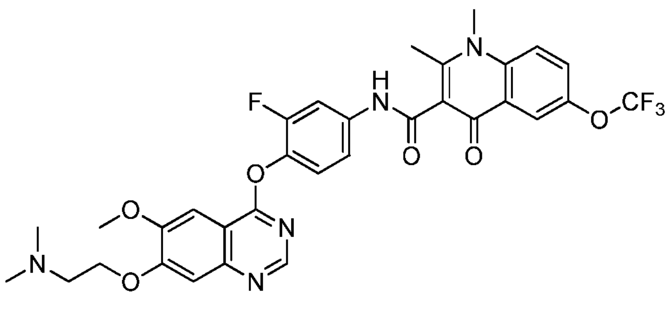

The synthesis method is referred to Example 1.

1H NMR (400 MHz, Chloroform-d) δ 12.30 (s, 1H), 8.62 (s, 1H), 8.35 (d, J = 2.3 Hz, 1H), 7.98 (dd, J = 12.3, 2.4 Hz, 1H), 7.66 (d, J = 9.3 Hz, 1H), 7.63 - 7.54 (m, 2H), 7.46 - 7.39 (m, 1H), 7.32 (s, 1H), 7.29 (d, J = 8.5 Hz, 1H), 4.31 (t, J = 5.9 Hz, 2H), 4.04 (s, 3H), 3.94 (s, 3H), 3.09 (s, 3H), 2.92 (t, J = 5.8 Hz, 2H), 2.41 (s, 6H).

Example 14: Preparation of *N*-(3-fluoro-4-((7-(isopentyloxy)-6-methoxyquinazolin-4-yl)oxy)phenyl)-1,2-dimethyl-4-oxo-6-(trif luoromethoxy)-1,4-dihydroquinoline-3-carboxamide (named as **TL230)**

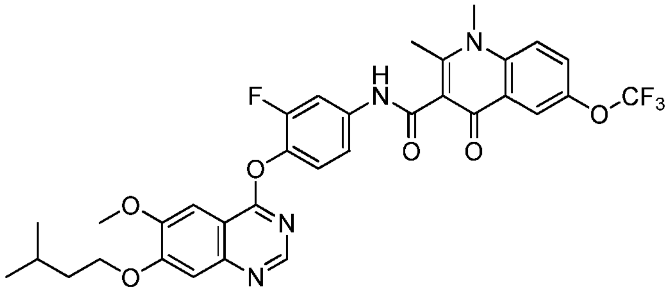

The synthesis method is referred to Example 1.

1H NMR (400 MHz, Chloroform-d) δ 12.28 (s, 1H), 8.61 (s, 1H), 8.37 - 8.31 (m, 1H), 7.97 (dd, J = 12.4, 2.5 Hz, 1H), 7.65 (d, J = 9.3 Hz, 1H), 7.58 (d, J = 12.1 Hz, 2H), 7.42 (ddd, J = 8.8, 2.5, 1.2 Hz, 1H), 7.32 (s, 1H), 7.30 - 7.24 (m, 1H), 4.24 (t, J = 6.7 Hz, 2H), 4.05 (s, 3H), 3.93 (s, 3H), 3.08 (s, 3H), 1.94 - 1.81 (m, 2H), 1.66 (s, 1H), 1.01 (s, 3H), 1.00 (s, 3H).

Example 15: Preparation of *N*-(3-fluoro-4-((6-methoxy-7-propoxyquinazolin-4-yl)oxy)phenyl)-1,2-dimethyl-4-oxo-6-(trifluoro methoxy)-1,4-dihydroquinoline-3-carboxamide (named as **TL240)**

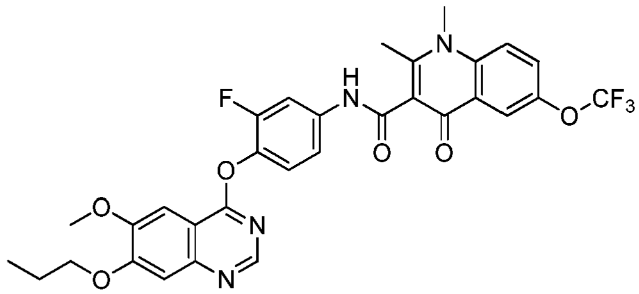

The synthesis method is referred to Example 1.

1H NMR (400 MHz, Chloroform-d) δ 12.26 (s, 1H), 8.61 (s, 1H), 8.31 (t, J = 1.8 Hz, 1H), 7.97 (dd, J = 12.4, 2.4 Hz, 1H), 7.63 (d, J = 9.4 Hz, 1H), 7.57 (d, J = 6.2 Hz, 2H), 7.41 (ddd, J = 8.8, 2.5, 1.2 Hz, 1H), 7.30 (s, 1H), 7.29 - 7.23 (m, 1H), 4.17 (t, J = 6.8 Hz, 2H), 4.05 (s, 3H), 3.91 (s, 3H), 3.06 (s, 3H), 1.98 (h, J = 7.2 Hz, 2H), 1.10 (t, J = 7.4 Hz, 3H).

Example 16: Preparation of *N*-(4-((7-ethoxy-6-methoxyquinazolin-4-yl)oxy)-3-fluorophenyl)-1,2-dimethyl-4-oxo-6-(trifluorom ethoxy)-1,4-dihydroquinoline-3-carboxamide (named as **TL241)**

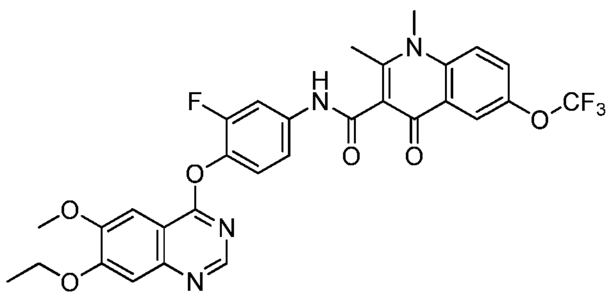

The synthesis method is referred to Example 1.

1H NMR (400 MHz, DMSO-d6) δ 10.78 (s, 1H), 8.55 (s, 1H), 8.12 - 8.03 (m, 2H), 7.94 (dd, J = 12.9, 2.3 Hz, 1H), 7.82 (dd, J = 9.3, 3.0 Hz, 1H), 7.59 (s, 1H), 7.50 (dd, J = 8.9, 2.3 Hz, 1H), 7.45 (t, J = 8.6 Hz, 1H), 7.39 (s, 1H), 4.27 (q, J = 6.9 Hz, 2H), 4.00 (s, 3H), 3.88 (s, 3H), 3.32 (s, 2H), 2.63 (s, 3H), 1.44 (t, J = 6.9 Hz, 3H).

Example 17: Preparation of *N*-(3-fluoro-4-((7-methoxy-6-(3-morpholinopropoxy)quinazolin-4-yl)oxy)phenyl)-1,2-dimethyl-4-oxo-6-(trifluoromethoxy)-1,4-dihydroquinoline-3-carboxamide (named as **TL236)**

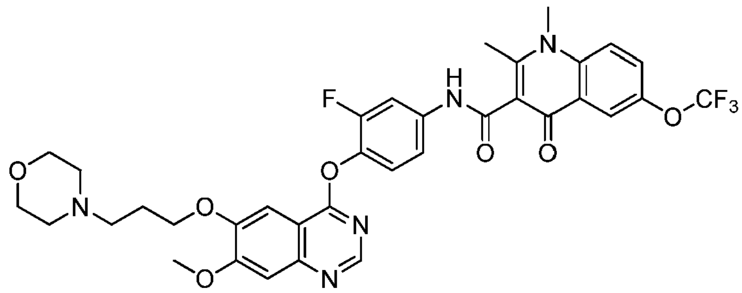

The synthesis method is referred to Example 1.

1H NMR (400 MHz, Chloroform-d) δ 12.33 (s, 1H), 8.62 (s, 1H), 8.41 - 8.34 (m, 1H), 7.99 (dd, J = 12.4, 2.4 Hz, 1H), 7.67 (d, J = 9.4 Hz, 1H), 7.60 (d, J = 8.6 Hz, 2H), 7.47 - 7.39 (m, 1H), 7.32 (s, 1H), 7.29 (d, J = 8.5 Hz, 1H), 4.29 (t, J = 6.6 Hz, 2H), 4.04 (s, 3H), 3.95 (s, 3H), 3.73 (t, J = 4.7 Hz, 5H), 3.10 (s, 3H), 2.59 (t, J = 7.1 Hz, 2H), 2.50 (t, J = 4.6 Hz, 4H), 2.14 (p, J = 6.8 Hz, 2H).

Example 18: Preparation of *N*-(3-fluoro-4-((6-methoxy-7-(3-morpholinopropoxy)quinolin-4-yl)oxy)phenyl)-1,2-dimethyl-4-oxo -6-(trifluoromethoxy)-1,4-dihydroquinoline-3-carboxamide (named as **CCB-310)** c

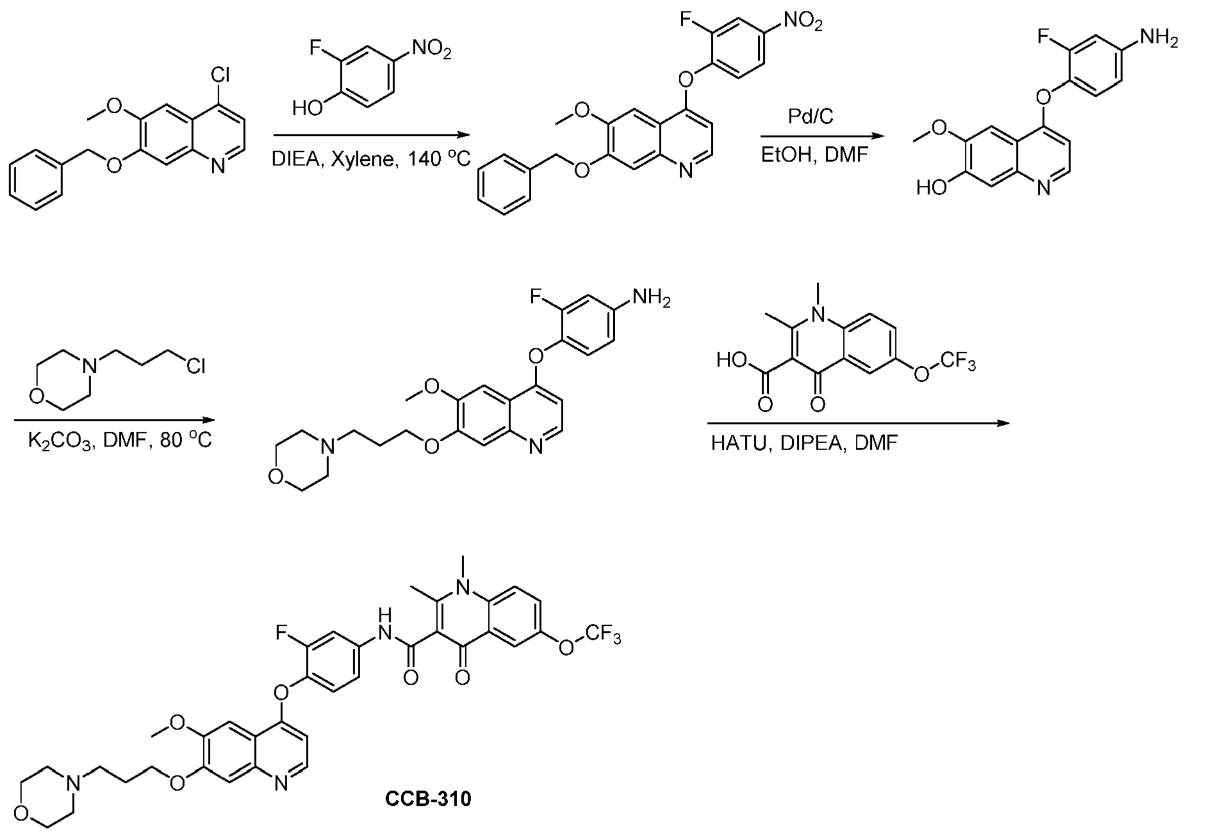


**CCB-310**

Step c1: Preparation of 7-(benzyloxy)-4-(2-fluoro-4-nitrophenoxy)-6-methoxyquinoline

To a reaction flask were added 7-benzyloxy-4-chloro-6-methoxyquinoline (4.5 g, 15 mmol), 2-fluoro-4-nitrophenol (2.4 g, 15 mmol), DIEA (18 mL) and xylene (9 mL), the mixture was heated to 140 °C and reacted overnight, then cooled to room temperature. A solid was precipitated, then the mixture was filtered, washed with ethanol, and then suction-dried to give 3.7 g (81.0%) of a white solid. $^1$HNMR (300 MHz, d6-DMSO) δ 8.56 (d, J = 5.1 Hz, 1H), 8.45 (dd, J = 10.5, 2.5 Hz, 1H), 8.20 (m, 1H), 7.61 (dd, J = 8.8, 8.8 Hz, 1H), 7.56 (s, 1H), 7.53 (m, 2H), 7.48 (s, 1H), 7.32-7.47 (m, 3H), 6.78 (d, J = 5.1 Hz, 1H), 5.33 (s, 2H), 3.93 (s, 3H)。MS (ESI), m/z: 421[M+H]+.

Step c2: Preparation of 4-(4-amino-2-fluorophenoxy)-6-methoxyquinolin-7-ol

7-(benzyloxy)-4-(2-fluoro-4-nitrophenoxy)-6-methoxyquinoline (3.0 g, 10 mmol) was dissolved in 10 mL of DMF, then 10% Pd/C (0.5 g) and 10 mL of ethanol were added, and the mixture was reacted at room temperature under a hydrogen atmosphere overnight. Pd/C was removed by filtration, and the filtrate was concentrated and subjected to a column to give 1.8 g (60%) of 4-(4-amino-2-fluorophenoxy)-6-methoxyquinolin-7-ol. $^1$H NMR (400 MHz, $d_6$-DMSO) δ 10.11 (s, 1H), 8.39 (d, J = 2.2 Hz, 1H), 7.49 (s, 1H), 7.27 (s, 1H), 7.06 (s, 1H), 6.68 - 6.40 (m, 2H), 6.32 (s, 1H), 5.49 (s, 2H), 3.95 (s, 3H). MS (ESI), m/z: 301 $[M+H]^+$.

Step c3: Preparation of 3-fluoro-4-((6-methoxy-7-(3-morpholinopropoxy)quinolin-4-yl)oxy)aniline

4-(4-amino-2-fluorophenoxy)-6-methoxyquinolin-7-ol (600 mg, 2 mmol), 4-(3-chloropropyl)morpholine (3- 5a) (982 mg, 6 mmol) and potassium carbonate (828 mg, 6 mmol) were added to DMF (20 mL), the mixture was heated to 80 °C and reacted for two hours, and then extracted three times with ethyl acetate. The organic phases were combined, washed with saturated brine, and dried by rotary evaporation, and then subjected to a column to give 615 mg (72%) of 3-fluoro-4-((6-methoxy-7-(3-morpholinopropoxy)quinolin-4-yl)oxy)aniline. $^1$H NMR (300 MHz, $d_6$-DMSO) $\delta$ 8.44 (d, J = 5.2 Hz, 1H), 7.50 (s, 1H), 7.37 (s, 1H), 7.06 (dd, J = 9.2, 8.8 Hz, 1H), 6.55 (dd, J = 13.2, 2.4 Hz, 1H), 6.47 (m, 1H), 6.38 (dd, J = 5.2, 1.0 Hz, 1H), 5.46 (s, 2H), 4.19 (t, J = 6.4 Hz, 2H), 3.94 (s, 3H), 3.59 (m, 4H), 2.47 (t, J = 7.1 Hz, S48 2H), 2.39 (m, 4H), 1.97 (m, 2H). MS (ESI), m/z: 428 $[M+H]^+$.

Step c4: Preparation of *N*-(3-fluoro-4-((6-methoxy-7-(3-morpholinopropoxy)quinolin-4-yl)oxy)phenyl)-1,2-dimethyl-4-oxo -6-(trifluoromethoxy)-1,4-dihydroquinoline-3-carboxamide (named as CCB-310)

3-fluoro-4-((6-methoxy-7-(3-morpholinopropoxy)quinolin-4-yl)oxy)aniline (428 mg, 1 mmol), 1,2-dimethyl-4-oxo-6-(trifluoromethoxy)-1,4-dihydroquinoline-3-carboxylic acid (277 mg, 1.2 mmol), HATU (570 mg, 1.5 mmol) and DIEA (0.5 mL, 3 mmol) were dissolved in 5 mL of DMF, and then the mixture was stirred overnight at room temperature. Ice water was added to the reaction solution, then a solid precipitated. The mixture was filtered, and the solid was extracted twice with dichloromethane. The organic phases were combined and washed once with saturated brine, dried over anhydrous $Na_2SO_4$, then filtered and dried by rotary evaporation, and then subjected to column chromatography to give 533 mg (75%) of a white solid.

$^1$H NMR (400 MHz, $d_6$-DMSO) δ 10.82 (s, 1H), 8.47 (d, J = 5.2 Hz, 1H), 8.08 (t, J = 6.2 Hz, 2H), 8.01 (dd, J = 13.1, 2.3 Hz, 1H), 7.83 (dd, J = 9.3, 2.8 Hz, 1H), 7.54 (d, J = 4.2 Hz, 2H), 7.45 (m, 2H), 6.49 (d, J = 5.0 Hz, 1H), 4.21 (t, J = 6.4 Hz, 2H), 3.96 (s, 3H), 3.88 (s, 3H), 3.69 - 3.50 (m, 4H), 2.63 (s, 3H), 2.47 (s, 2H), 2.41 (s, 4H), 2.05 - 1.91 (m, 2H).. MS (ESI), m/z: 711 $[M+H]^+$.

Comparative Example 1: Preparation of 6-ethyl-*N*-(3-fluoro-4-((6-methoxy-7-(3-morpholinopropoxy)quinazolin-4-yl)oxy)phenyl)-1,2-dime thyl-4-oxo-1,4-dihydroquinoline-3-carboxamide (named as **GDL5000123)**

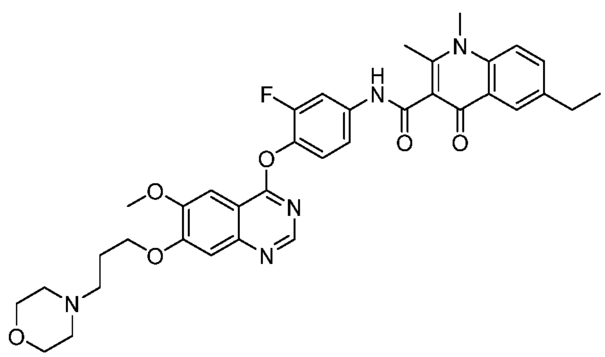

The synthesis method is referred to Example 1.

$^1$H NMR (500 MHz, $d_6$-DMSO) $\delta$ 11.01 (s, 1 H), 8.56 (s, 1 H), 8.08 (d, *J* = 1.5 Hz, 1 H), 7.97-7.94 (dd, J= 2.0, 13.0 Hz, 1 H), 7.81 (d, *J* = 9.0 Hz, 1 H), 7.67-7.65 (dd, *J* = 2.0, 8.5 Hz, 1 H), 7.58 (s, 1 H), 7.50 (m, 1 H), 7.44 (t, *J* = 9.0 Hz, 1 H), 7.40 (s, 1 H), 4.26 (t, *J* = 6.0 Hz, 2 H), 3.99 (s, 3 H), 3.83 (s, 3 H), 3.60 (s, 4 H), 2.77 (q, *J* = 7.5 Hz, 2 H), 2.65 (s, 3 H), 2.50 (m, 2 H), 2.41 (s, 4 H), 1.99 (t, *J* = 6.5 Hz, 2 H), 1.25 (t, *J* = 7.5 Hz, 2 H). MS (ESI), m/z: 656[M+H]+.

Example 19: $IC_{50}$ tests for quinoline and quinazoline compounds against AXL kinase

Detection of the activity of kinase: Enzyme-linked immunosorbent assay (ELISA) technology was used to detect the inhibitory activities of the compounds against the kinase. The enzyme reaction substrate Poly(Glu, Tyr) 4:1 was diluted to 20 $\mu$g/mL with potassium-free PBS (10 mM sodium phosphate buffer, 150 mM NaCl, pH 7.2-7.4), and the enzyme label plate was coated with 125 $\mu$L/well, then incubated at 37°C for 12-16 hours. After the liquid in the well was discarded, the plate was washed three times with T-PBS (PBS containing 0.1% Tween-20), 200 $\mu$L T-PBS per well, 5 minutes for each time. The enzyme label plate was dried in an oven at 37 °C for 1-2 hours. To each well was added 50 $\mu$L of ATP solution diluted with reaction buffer (50 mM HEPES, pH 7.4, 50 mM $MgCl_2$, 0.5 mM $MnCl_2$, 0.2 mM $Na_3VO_4$, 1 mM DTT) with a final concentration of 5 $\mu$M. The test compounds were diluted with DMSO to an appropriate concentration, as 1 $\mu$L/well or containing the corresponding concentration of DMSO (negative control well), and then the AXL kinase domain recombinant protein (eurofins, 14-512) diluted with 49 $\mu$L of reaction buffer was added to initiate the reaction. Two wells with no enzyme for control are required for each experiment. The plate was incubated on a shaker (100 rpm) at 37 °C and reacted for 1 hour. The plate was washed three times with T-PBS. 100 $\mu$L/well of primary antibody PY99 dilution was added and the plate was reacted in a shaker at 37 °C for 0.5 hour. The plate was washed three times with T-PBS. 100 $\mu$L/well of secondary antibody horseradish peroxidase-labeled goat anti-mouse IgG dilution was added and the plate was reacted in a shaker at 37 °C for 0.5 hour. The plate was washed three times with T-PBS. 2 mg/ml of OPD color solution was added with 100 $\mu$L/well (diluted with 0.1 M citric acid-sodium citrate buffer (pH=5.4) containing 0.03% $H_2O_2$), and the plate was reacted at 25 °C in the dark for 1-10 minutes. (Ultrasound is required for the dissolution of OPD, and the color solution needs to be prepared for immediate use). 50 $\mu$L/well of 2 M $H_2SO_4$ was added to stop the reaction, and the wavelength-adjustable enzyme-labeling instrument SPECTRA MAX 190 was used to read the data at a wavelength of 490 nm.

The inhibition rate of the sample was obtained by the following formula:

$$\text{Inhibition rate (\%) of sample} = \left(1 - \frac{\text{OD value of compound - OD value of control well without enzyme}}{\text{OD value of negative control well - OD value of control well without enzyme}}\right) \times 100$$

The $IC_{50}$ values were obtained by regression with the four-parameter method using the software attached to the enzyme-labeling instrument.

In the competition experiments of quinoline and quinazoline compounds with ATP, all compounds exhibited strong inhibitory activities against AXL kinase (the results are shown in Table 1). As for the modification of $R_1$ and $R_2$ substituents in the general formula (I), it has been found that when $R_1$ and $R_2$ are hydrophilic substituents, the compounds have better inhibitory activities and can tolerate larger modifications of the substituents. Compounds R428 and AC220 are reference examples for comparative purposes.

Table 1 - Number of the compounds and corresponding results of the inhibitory activities against the kinase.

| Compds | AXL $IC_{50}$ (nM) |
|---|---|
| TL-209 | 2.0 |
| TL-212 | 1.5 |
| TL-199 | 1.8 |
| TL-213 | 2.2 |
| TL-197 | 4.2 |
| TL-198 | 2.1 |
| TL-204 | 1.6 |
| TL-134 | 1.1 |
| CCB-310 | 1.8 |

(continued)

| Compds | AXL $IC_{50}$ (nM) |
|---|---|
| GDL5000123 | 1.2 |
| R428 | 4.2 |

Example 20: $IC_{50}$ tests for quinoline and quinazoline compounds against AXL kinase (Z'-LYTE™ technology)

Detection of the activity of kinase: the inhibitory activities of the compounds against AXL kinase (eurofins, 14-500) were detected through a second-order reaction by the Z'-LYTE™ technology (detected by fluorescence, enzyme-coupled form, based on the difference in a sensitivity of phosphorylated and non-phosphorylated polypeptides to proteolytic cleavage), based on the principle of fluorescence resonance energy transfer (FRET), using Z'-LYTE™ FRET peptide substrate (Z'-LYTE™ Tyrosine 6 Peptide Substrate, Invitrogen, PV4122).

Enzymatic reaction: To a 384-well plate was added 5 μL of enzyme-substrate system (50 mM HEPES, pH 6.5, 0.01% BRIJ-35, 10 mM $MgCl_2$, 1 mM EGTA, 0.02% $NaN_3$), and 5 nL of a compound (concentration gradient) was transferred thereto using an echo520 ultramicro-liquid pipetting system. After the plate was shaken at room temperature for 10-20 min, 25 nL of ATP (a final concentration of 50 μM) was transferred thereto using the echo520 ultramicro-liquid pipetting system. After shaking and well mixing, the mixture was centrifuged and reacted at 30 °C in the dark for 1.5 h.

Detecting reaction: 2.5 μL of Development Solution (1:128 dilution) was added to each well and the plate was incubated at 37 °C in the dark for 1 h, then 5 μL of Stop Reagent was added.

Plate reading: fluorescent signals were detected using Perkin Elmer EnVision Multimode Plate Reader (excitation wavelength is 400 nm, emission wavelengths are 460 nm, 535 nm).

Calculation: the inhibition rate of each well was calculated from the fully active wells and the control signal wells. The data analysis method is as follows:

$$\text{Phosphorylation ratio} = 1 - \{(\text{emission ratio} \times \text{F100\%} - \text{C100\%}) / [\text{C0\%} - \text{C 100\%} + \text{emission ratio} \times (\text{F100\%} - \text{F0\%})]\} \times 100;$$

$$\text{Inhibition rate} = 100 \times (1 - \text{compound phosphorylation ratio} / \text{negative control phosphorylation ratio}).$$

The $IC_{50}$ values were calculated by GraphPad Prism software.

In the competition experiments of quinoline and quinazoline compounds with ATP, all compounds exhibited strong inhibitory activities against AXL kinase (the results are shown in Table 2). As for the modification of $R_1$ and $R_2$ substituents in the general formula (I), it has been found that when $R_1$ and $R_2$ are hydrophilic substituents, the compounds have better inhibitory activities and can tolerate larger modifications of the substituents.

Table 2 - Number of the compounds and corresponding results of the inhibitory activities against the kinase.

| Compds | AXL $IC_{50}$ (nM) |
|---|---|
| TL-216 | 1.5 |
| TL-226 | 4.7 |
| TL-230 | 59.4 |
| TL-231 | 2.3 |
| TL-233 | 1.9 |
| TL-236 | 2.4 |
| TL-23 8 | 1.2 |
| TL-240 | 6.1 |
| TL-241 | 2.6 |
| R428 | 5.5 |

Example 21: $IC_{50}$ tests for quinoline and quinazoline compounds against Flt3 kinase

Detection of the activity of kinase: the inhibitory activities of the compounds against Flt3 kinase (life, PV6253) were detected through a second-order reaction by the Z'-LYTE™ technology (detected by fluorescence, enzyme-coupled form, based on the difference in a sensitivity of phosphorylated and non-phosphorylated polypeptides to proteolytic cleavage), based on the principle of fluorescence resonance energy transfer (FRET), using Z'-LYTE™ FRET peptide substrate (Z'-LYTE™ Tyrosine 2 Peptide Substrate, Invitrogen, PV3191).

Enzymatic reaction: To a 384-well plate were added 5 μL of enzyme-substrate system (50 mM HEPES, pH 7.5, 0.01% BRIJ-35, 10 mM $MgCl_2$, 1 mM EGTA), 2.5 μL of a compound (concentration gradient) and 2.5 μL of a mixed solution of ATP (a final concentration of a substrate Z'-LYTE™ Tyrosine 2 Peptide Substrate was 2 μM, and a final concentration of ATP was 500 μM), and then the plate was incubated at 37 °C in the dark for 1 h.

Detecting reaction: 5 μL of Development Solution (1:64 dilution) was added to each well and the plate was incubated at 37 °C in the dark for 1 h, then 5 μL of Stop Reagent was added.

Plate reading: fluorescent signals were detected using Synergy HI Microplate Reader (excitation wavelength is 400 nm, emission wavelength is 445 nm, 535 nm).

Calculation: the inhibition rate of each well was calculated from the fully active wells and the control signal wells. The data analysis method is as follows:

$$\text{Phosphorylation ratio} = 1 - \{(\text{emission ratio} \times F100\% - C100\%) / [C0\% - C\ 100\% + \text{emission ratio} \times (F100\% - F0\%)]\} \times 100;$$

$$\text{Inhibition rate} = 100 \times (1 - \text{compound phosphorylation ratio} / \text{negative control phosphorylation ratio}).$$

The $IC_{50}$ values were calculated by GraphPad Prism software.

In the competition experiments of quinoline and quinazoline compounds with ATP, all compounds exhibited strong inhibitory activities against Flt3 kinase (the results are shown in Table 3). As for the modification of $R_1$ and $R_2$ substituents in the general formula (I), it has been found that when $R_1$ and $R_2$ are hydrophilic substituents, the compounds have better inhibitory activities and can tolerate larger modifications of the substituents.

Table 3 - Number of the compounds and corresponding results of the inhibitory activities against the kinase.

| Compds | Flt3 $IC_{50}$ (nM) |
|---|---|
| TL-209 | 9.2 |
| TL-212 | 5.2 |
| TL-199 | 25.8 |
| TL-213 | 6.4 |
| TL-197 | 4.7 |
| TL-198 | 2.3 |
| TL-204 | 11.2 |
| TL-134 | 1.9 |
| CCB-310 | 9.5 |
| GDL5000123 | 10.0 |
| AC220 | 10.3 |

Example 22: Effects of quinoline and quinazoline compounds on the proliferation of MV4-11 cells

The inhibitory effects of the compounds on the proliferation of MV4-11 cells were detected by a CCK-8 cell counting kit (Dojindo). The specific steps are as follows: MV4-11 cells in the logarithmic phase were seeded into a 96-well culture plate at a suitable density for 90 μL per well. After overnight cultivation, the compounds at different concentrations were added to act for 72 hr, and a solvent control group was set (negative control). After 72 h of the compounds

acting on the cells, the effects of the compounds on cell proliferation were detected using the CCK-8 cell counting kit (Dojindo). 10 μL of CCK-8 reagent was added to each well and the plate was placed in an incubator at 37 °C for 2-4 hours. After that, a full-wavelength microplate enzyme-labeling instrument SpectraMax 190 was used to read with a measurement wavelength of 450 nm.
The inhibition rate (%) of the compound on tumor cell growth was calculated by the following formula:

$$\text{Inhibition rate (\%)} = (\text{OD control well - OD administration well}) / \text{OD control well} \times 100\%$$

The $IC_{50}$ values were obtained by regression with the four-parameter method using the software attached to the enzyme-labeling instrument.
The quinoline and quinazoline compounds exhibited strong inhibitory activities against leukemia MV4-11 cells (the results are shown in Table 4).

Table 4 - $IC_{50}$ values of the inhibitions of the compounds against the proliferation of MV4-11 cells.

| Compds | $IC_{50}$ (nM) |
|---|---|
| TL-134 | <0.4 |
| AC220 | <4 |

Example 23: A comparative study on the species of metabolism of quinoline and quinazoline compounds in hepatocytes

In this example, a UPLC-UV/Q-TOF MS method was used to evaluate the differences in the metabolic processes of TL134 in hepatocytes of five species of human, monkey, canine, rat, and mouse. It provides a reference for the selection of preclinical pharmacokinetics and studies on animal species safety evaluation.
The materials and methods are as follows:

1. Medicines and Reagents

| | |
|---|---|
| TL134 | Preparation in the Example 1 |
| Mixed primary human hepatocytes (Lot No. 1410266) | Xenotech Company, USA |
| Mixed primary macaca fascicularis hepatocytes (Lot No. IXCH) | RILD Company |
| Mixed primary male beagle canine hepatocytes (Lot No. 1410275) | Xenotech Company, USA |
| Mixed primary male SD rat hepatocytes (Lot No. 1210260) | Xenotech Company, USA |
| Mixed primary male CD-1 mouse hepatocytes (Lot No. 1510134) | Xenotech Company, USA |
| Ammonium acetate (chromatographically pure) | ROE Company, USA |
| Acetonitrile (chromatographically pure) | Merck Company, Germany |
| Formic acid (chromatographically pure) | Fluka Company, Germany |

2. Incubation Systems for In Vitro Metabolism Studies

The total volume of each incubation sample was 100 μL, the medium was WME medium (pH 7.4), and the incubation sample included hepatocytes with a cell density of $1.0 \times 10^6$ cells/mL and TL134 with a final concentration of 3.0 μM; the negative control sample was incubated with thermal-inactivated mixed hepatocytes (without cell counting) of five species of animals and TL134, and the buffer control sample was incubated with WME medium and TL134. All samples were incubated at 37 °C for 180 min, and then 100 μL of ice-cold acetonitrile was added to stop the reaction. The samples were stored at -70 °C for ready to be tested, and all incubation samples were double samples.

3. Instruments and Conditions

Triple TOF $5600^+$ quadrupole-time-of-flight tandem mass spectrometer (Q-TOF MS), equipped with electrospray ionization source (ESI source) and CDS automatic calibration system, AB SCIEX Company, USA; Acquity UPLC liquid chromatography system, including binary infusion pump, autosampler, column oven, degasser and TUV ultraviolet detector, Waters Company, USA.

Chromatographic conditions: the chromatographic column was ACQUITY™ HSS T3 C18 column (100 × 2.1 mm I.D., 1.8 μm particle size), Waters Company, USA; column temperature was 40 °C; flow rate was 0.4 mL/min; UV detection wavelength was 254 nm; mobile phase gradient is shown in the table below.

| Time (min) | A (5 mM ammonium acetate containing 0.1% formic acid, %) | B (acetonitrile, %) |
|---|---|---|
| 0 | 90 | 10 |
| 1 | 90 | 10 |
| 8.5 | 46 | 54 |
| 9.5 | 5 | 95 |
| 10.5 | 5 | 95 |
| 11.5 | 90 | 10 |
| 15 | 90 | 10 |

Mass spectrometry conditions: electrospray ionization source (ESI), detecting with positive ion scanning (high sensitivity mode) manner, GAS1: 55 psi, GAS2: 50 psi, Curtain GAS: 40 psi, source temperature was 500 °C, ion spray voltage (ISVF) was 5500 V with a declustering voltage of 80 V, the collision energy was 10 eV during first-level full scanning, and the collision energy was 20 ± 10 eV during product ion scanning, scanning range was m/z 80-1000, automatic calibration system (CDS) with external standard method was used for mass number correction.

4. Sample Pretreatment

The double samples of the hepatocyte incubation solution of each species were full taken and combined, vortex mixed for 1 min and centrifuged for 5 min (14,000 rpm), then all the supernatant was taken, transferred to a 10 mL test tube, and dried under a nitrogen flow at 40 °C. Then the residue was dissolved in 150 μL of acetonitrile-water (10 : 90, v/v), centrifuged for 5 min (14,000 rpm), and 5.0 μL of the supernatant was taken for UPLC-UV/Q-TOF MS analysis. The negative control samples and buffer control samples were processed in the same way as hepatocyte incubation samples.

5. Data Analysis

The softwares of Analyst ®TF V1.6 from AB Sciex Company and Masslynx V4.1 from Waters Company were used for data acquisition, and the softwares of PeakView ® V1.2 and MetabolitePilot V1.5 from AB Sciex Company were used for data analysis.

6. Experimental Results

The data of hepatocytes incubation fluids of TL134 in human, monkey, canine, rat and mouse were processed using MetabolitePilot software to obtain the related metabolites spectrums (FIG. 1 and FIG.2), and the ultraviolet chromatograms are shown in FIG.3 and FIG.4. The metabolites are named in order of their mass-to-charge ratio from small to large, and the metabolites with the same mass-to-charge ratio are named in order of the chromatographic retention times from front to rear, the UPLC-UV/Q-TOF MS informations of TL134 and metabolites in hepatocyte incubation systems are shown in Table 5.

The metabolisms of compound GDL5000123 in hepatocytes of both monkey and canine species were evaluated by the same method and conditions as described above. The results are shown in Table 6.

Table 5 - UPLC-UV/Q-TOF MS related informations of the metabolites of TL134 in hepatocytes of five species of human, monkey, canine, rat and mouse

| | Metabolic Pathway | Mass-to -Charge Ratio | Molecular Formula | Mass Deviat ion (ppm) | Reten tion Time (min) | LC-MS Peak Area (×10³) | | | | | |
|---|---|---|---|---|---|---|---|---|---|---|---|
| | | | | | | Ther mal-i nactiv ated mixed hepat ocyte s | Hum an | Mon key | Cani ne | Rat | Mouse |
| M0 | prototype drug | 712.2409 | $C_{35}H_{33}N_5O_7F_4$ | 2.8 | 7.37 | 1750 | 708 | 920 | 832 | 1130 | 1970 |
| M1 | amide bond hydrolysis (acid) | 302.0639 | $C_{13}H_{10}NO_4F_3$ | 1.3 | 7.53 | | 19.2 | 8.32 | 7.95 | 13.1 | 10.4 |
| M2 | *O*-dealkyl ation | 411.0965 | $C_{19}H_{15}N_2O_4F_4$ | 0.6 | 7.19 | | 16.9 | 6.71 | 5.16 | 12.3 | 16.2 |
| M3 | amide bond hydrolysis (acid) | 429.1937 | $C_{22}H_{25}N_4O_4F$ | 1.0 | 4.38 | | 7.93 | 2.80 | 2.34 | 3.70 | 6.25 |
| M4 | *O*-depropy lmorpholi ne ring | 585.1398 | $C_{28}H_{20}N_4O_6F_4$ | 1.1 | 8.73 | | 33.2 | 10.5 | 18.5 | 20.6 | 9.03 |
| M5 | oxidized-d emorpholine ring (acid) | 657.1592 | $C_{31}H_{24}N_4O_8F_4$ | -1.7 | 8.74 | | 0.06 | 3.64 | 21.7 | 1.91 | 0.03 |
| M6 | *N*-demeth ylation | 698.2249 | $C_{34}H_{31}N_5O_7F_4$ | 2.3 | 8.16 | | | | | | 1.23 |
| M7 | mono-oxi dative dehydroge nation | 726.2188 | $C_{35}H_{31}N_5O_8F_4$ | 0.9 | 8.98 | | | | | 2.11 | 2.57 |
| M8 | mono-oxidation | 728.2345 | $C_{35}H_{33}N_5O_8F_4$ | 1.0 | 7.49 | | 198 | 19.1 | 99.7 | 235 | 35.5 |
| | | | | | | UV Chromatographic Peak Area | | | | | |
| | | | | | | Ther mal-I nactivated Mixed Hepat ocyte s | Human | Monkey | Canine | Rat | Mouse |
| M0 | prototype | | | | 7.32 | 467 | 406 | 270 | 236 | 315 | 577 |
| M4/M5 | *O*-depropylmorpholine ring/oxidized-demorpholine ring (acid) | | | | 8.66 | | * | * | * | * | |

(continued)

| | | | UV Chromatographic Peak Area | | | | | |
|---|---|---|---|---|---|---|---|---|
| | | | Ther mal-I nactivated Mixed Hepat ocyte s | Human | Monkey | Canine | Rat | Mouse |
| M8 | mono-oxidat ion | 7.43 | | 88 | 13 | 43 | 78 | 18 |

*: The related metabolites were detected, but the UV peak areas thereof cannot be accurately integrated due to matrix interference

Table 6 - UPLC/Q-TOF MS related informations of the metabolites of GDL5000123 in hepatocytes of both species of monkey and canine

| | Metabolic Pathway | Mass-to-C harge Ratio | Formula | Mass Molecular Deviation (ppm) | Retention Time (min) | LC-MS Peak Area ($\times 10^3$) | | |
|---|---|---|---|---|---|---|---|---|
| | | | | | | inactivated | Monkey | Canine |
| M0 | prototype drug | 656.2889 | $C_{36}H_{38}N_5O_6F$ | 1.5 | 8.07 | 166 | 264 | 218 |
| M1 | oxidized-demorp holine ring | 601.2078 | $C_{32}H_{29}N_4O_7F$ | -2.4 | 9.43 | | 2.57 | 5.84 |
| M2 | mono-oxidation | 617.2042 | $C_{32}H_{29}N_4O_8F$ | 0 | 7.33 | | 1.19 | 3.42 |
| M3 | dehydrogenation | 654.2721 | $C_{36}H_{36}N_5O_6F$ | -0.2 | 7.74 | | 1.35 | 1.01 |
| M4 | mono-oxidative dehydrogenation | 670.2673 | $C_{36}H_{36}N_5O_7F$ | 0.2 | 6.41 | | 1.76 | 0.92 |
| M5-1 | mono-oxidation | 672.2835 | $C_{36}H_{38}N_5O_7F$ | 1.0 | 5.96 | | 25.8 | |
| M5-2 | mono-oxidation | 672.2839 | $C_{36}H_{38}N_5O_7F$ | 1.6 | 6.03 | | 22.4 | 23.3 |
| M6-1 | dual-oxidation | 688.2780 | $C_{36}H_{38}N_5O_8F$ | 0.5 | 5.20 | | 1.41 | |
| M6-2 | dual-oxidation | 688.2778 | $C_{36}H_{38}N_5O_8F$ | 0.2 | 6.19 | | | 2.26 |
| M8 | dual-oxidative dehydrogenation | 686.2623 | $C_{36}H_{36}N_5O_8F$ | 0.3 | 6.57 | | | 0.88 |
| | | | | | | UV Peak Area | | |
| | | | | | | inactivated | Monkey | Canine |
| M0 | prototype drug | | | | | 129 | 191 | 144 |
| M5-1 | mono-oxidation | | | | | | 102 | |
| M5-2 | mono-oxidation | | | | | | 32.3 | 53.7 |

Analysis of experimental data: judging from the UV chromatographic peak areas, after incubating the compound GDL5000123 with the hepatocytes of monkey and canine for 180 min, respectively, approximately 41.3% and 27.2% of the prototype drug in each incubation system had been metabolized, respectively; judging from the UV chromatographic peak areas, after incubating the compound TL134 with the hepatocytes of human, monkey, canine, rat and mouse for 180 min, approximately 17.8%, 4.6%, 15.4%, 19.8% and 3.0% of the prototype drug in each incubation system had been metabolized, respectively. It can be seen that, the introduction of trifluoromethoxy instead of ethyl in the molecular quinolone fragment can significantly improve the stability of the compound, improve the metabolic stability of the compound, and increase the exposure, thus can improve the in vivo drug efficacy of the compound, and can reduce the dosage with the same drug effect.

Each of the technical features of the above examples may be combined arbitrarily. To simplify the description, not all the possible combinations of each of the technical features in the above examples are described. However, all of the combinations of these technical features should be considered as within the scope of this disclosure, as long as such combinations do not contradict with each other.

The above-mentioned examples are merely illustrative of several embodiments of the present disclosure, which are described specifically and in detail, but it cannot be understood to limit the scope of the present disclosure. It should be noted that, for those ordinary skilled in the art, several variations and improvements may be made without departing from the concept of the present disclosure, and all of which are within the protection scope of the present disclosure. Therefore, the protection scope of the present disclosure shall be defined by the appended claims.

## Claims

1. A quinoline or quinazoline compound having a structure represented by a formula ( I ) or a pharmaceutically acceptable salt thereof, a stereoisomer thereof, or a deuterated analog thereof:

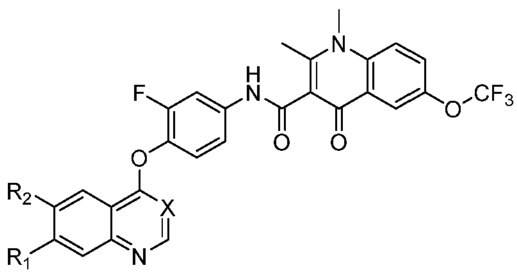

( I )

wherein, X is selected from CH and N;

$R_1$ and $R_2$ are each independently selected from a group consisting of hydrogen, halogen, $-(CR_4R_5)_OR_3$ and $-O(CR_4R_5)_OR_3$;
wherein, o is an integer from 0 to 6;
$R_3$, $R_4$ and $R_8$ are each independently selected from a group consisting of -H, $C_1$~$C_6$ alkyl, halogen, $-CF_3$, $-OCF_3$, $-(C=O)-NR_8R_9$, $-COOR_8$, -SOm-$NR_8R_9$, $-CHR_8R_9$, $-OR_8$ and $-NR_8R_9$;
$R_8$ and $R_9$ are each independently selected from a group consisting of hydrogen, halogen and $C_1$~$C_6$ alkyl, or, $R_8$ and $R_9$ together with N connected thereto form a saturated or unsaturated 5- to 8-membered heterocyclic group; wherein, the saturated or unsaturated 5- to 8-membered heterocyclic group can be independently and optionally substituted with one or more $R_{10}$; wherein $R_{10}$ is selected from a group consisting of $C_1$~$C_6$ alkyl;
or, $R_1$ and $R_2$ form a substituted or unsubstituted $C_5$~$C_{18}$ aliphatic cycloalkyl containing 1 to 4 heteroatoms.

2. The quinoline or quinazoline compound of claim 1 or a pharmaceutically acceptable salt thereof, a stereoisomer thereof, or a deuterated analog thereof, wherein $R_1$ and $R_2$ are each independently $-O(CR_4R_5)_OR_3$;

$R_3$, $R_4$ and $R_5$ are each independently selected from a group consisting of -H, $C_1$~$C_6$ alkyl, $-OR_8$ and $-NR_8R_9$;
$R_8$ and $R_9$ are each independently selected from a group consisting of $C_1$~$C_6$ alkyl, or, $R_8$ and $R_9$ together with N connected thereto form a saturated or unsaturated 5- to 8-membered heterocyclic group; wherein, the saturated or unsaturated 5- to 8-membered heterocyclic group can be independently and optionally substituted with one or more $R_{10}$; wherein $R_{10}$ is selected from a group consisting of $C_1$~$C_6$ alkyl.

**3.** The quinoline or quinazoline compound of claim 2 or a pharmaceutically acceptable salt thereof, a stereoisomer thereof, or a deuterated analog thereof, wherein $R_1$ is $-O(CH_2)_OR_3$;

o is an integer from 0 to 4; $R_3$ is selected from a group consisting of -H, $C_1$~$C_6$ alkyl, $C_1$~$C_3$ alkoxy and $-NR_8R_9$; $R_8$ and $R_9$ are each independently selected from a group consisting of $C_1$~$C_3$ alkyl, or $R_8$ and $R_9$ together with N connected thereto form a saturated or unsaturated 5- to 6-membered heterocyclic group; wherein, the saturated or unsaturated 5- to 6-membered heterocyclic group can be independently and optionally substituted with one or more $R_{10}$; wherein $R_{10}$ is selected from a group consisting of $C_1$~$C_3$ alkyl.

**4.** The quinoline or quinazoline compound of claim 3 or a pharmaceutically acceptable salt thereof, a stereoisomer thereof, or a deuterated analog thereof, wherein $R_1$ is selected from a group consisting of methoxyl, ethoxyl, propoxyl, 2-methoxyethoxyl, 3-methoxypropoxyl, 3-morpholinopropoxyl, 2-(pyrrolidin-1-yl)ethoxyl, 3-(pyrrolidin -1-yl)propoxyl, (piperidin-1-yl)ethoxyl, (piperidin-1-yl)propoxyl, 4-methoxybutoxyl, 2-morpholinoethoxyl, (4-methylpiperazin-1-yl)propoxyl, dimethylaminoethoxyl and isopentyloxyl.

**5.** The quinoline or quinazoline compound of claim 2 or a pharmaceutically acceptable salt thereof, a stereoisomer thereof, or a deuterated analog thereof, wherein $R_2$ is $-O(CH_2)_OR_3$;

o is an integer from 0 to 4;
$R_3$ is selected from a group consisting of: -H, $C_1$~$C_3$ alkyl, $C_1$~$C_3$ alkoxy and $-NR_8R_9$;
$R_8$ and $R_9$, together with N connected thereto, form a saturated 5- to 6-membered heterocyclic group.

**6.** The quinoline or quinazoline compound of claim 5 or a pharmaceutically acceptable salt thereof, a stereoisomer thereof, or a deuterated analog thereof, wherein $R_2$ is selected from a group consisting of methoxyl, ethoxyl, propoxyl, 2-methoxyethoxyl, 3-methoxypropoxyl, 2-morpholinoethoxyl and 3-morpholinopropoxyl.

**7.** The quinoline or quinazoline compound of any one of claims 1 to 6 or a pharmaceutically acceptable salt thereof, a stereoisomer thereof, or a deuterated analog thereof, wherein X is N.

**8.** The quinoline or quinazoline compound of claim 1 or a pharmaceutically acceptable salt thereof, a stereoisomer thereof, or a deuterated analog thereof, wherein the quinoline or quinazoline compound is selected from a group consisting of:

*N*-(3-fluoro-4-((6-methoxy-7-(3-morpholinopropoxy)quinazofin-4-yl)oxy)phenyl)-1,2-dimethy l-4-oxo-6-(trifluoromethoxy)-1,4-dihydroquinoline-3-carboxamide,
*N*-(4-((6,7-bis(2-methoxyethoxy)quinazolin-4-yl)oxy)-3-fluorophenyl)-1,2-dimethyl-4-oxo-6-( trifluoromethoxy)-1,4-dihydroquinoline-3-carboxamide,
*N*-(4-((6,7-dimethoxyquinazolin-4-yl)oxy)-3-fluorophenyl)-1,2-dimethyl-4-oxo-6-(trifluorome thoxy)-1,4-dihydroquinoline-3-carboxamide,
*N*-(3-fluoro-4-((7-methoxy-6-(3-methoxypropoxy)quinazolin-4-yl)oxy)phenyl)-1,2-dimethyl-4 -oxo-6-(trifluoromethoxy)-1,4-dihydroquinoline-3-carboxamide,
*N*-(3-fluoro-4-((6-methoxy-7-(4-methoxybutoxy)quinazolin-4-yl)oxy)phenyl)-1,2-dimethyl-4-oxo-6-(trifluoromethoxy)-1,4-dihydroquinoline-3-carboxamide,
*N*-(3-fluoro-4-((6-methoxy-7-(2-morpholinoethoxy)quinazolin-4-yl)oxy)phenyl)-1,2-dimethyl-4-oxo-6-(trifluoromethoxy)-1,4-dihydroquinoline-3-carboxamide,
*N*-(3-fluoro-4-((6-methoxy-7-(3-(pyrrolidin-1-yl)propoxy)quinazolin-4-yl)oxy)phenyl)-1,2-di methyl-4-oxo-6-(trifluoromethoxy)-1,4-dihydroquinoline-3-carboxamide,
*N*-(3-fluoro-4-((6-methoxy-7-(2-(piperidin-1-yl)ethoxy)quinazolin-4-yl)oxy)phenyl)-1,2-dimet hyl-4-oxo-6-(trifluoromethoxy)-1,4-dihydroquinoline-3-carboxamide,
*N*-(3-fluoro-4-((6-methoxy-7-(3-(4-methylpiperazin-1-yl)propoxy)quinazolin-4-yl)oxy)phenyl) -1,2-dimethyl-4-oxo-6-(trifluoromethoxy)-1,4-dihydroquinoline-3-carboxamide,
*N*-(3-fluoro-4-((6-methoxy-7-(2-(pyrrolidin-1-yl)ethoxy)quinazolin-4-yl)oxy)phenyl)-1,2-dime thyl-4-oxo-6-(trifluoromethoxy)-1,4-dihydroquinoline-3-carboxamide,
*N*-(3-fluoro-4-((6-methoxy-7-(3-methoxypropoxy)quinazolin-4-yl)oxy)phenyl)-1,2-dimethyl-4 -oxo-6-(trifluoromethoxy)-1,4-dihydroquinoline-3-carboxamide,
*N*-(3-fluoro-4-((6-methoxy-7-(3-(piperidin-1-yl)propoxy)quinazolin-4-yl)oxy)phenyl)-1,2-dim ethyl-4-oxo-6-(trifluoromethoxy)-1,4-dihydroquinoline-3-carboxamide,
*N*-(4-((7-(2-(dimethylamino)ethoxy)-6-methoxyquinazolin-4-yl)oxy)-3-fluorophenyl)-1,2-dim ethyl-4-oxo-6-(trifluoromethoxy)-1,4-dihydroquinoline-3-carboxamide,
*N*-(3-fluoro-4-((7-(isopentyloxy)-6-methoxyquinazolin-4-yl)oxy)phenyl)-1,2-dimethyl-4-oxo-6 -(trifluoromethoxy)-1,4-dihydroquinoline-3-carboxamide,
*N*-(3-fluoro-4-((6-methoxy-7-propoxyquinazolin-4-yl)oxy)phenyl)-1,2-dimethyl-4-oxo-6-(trifl uoromethoxy)-1,4-dihydroquinoline-3-carboxamide,
*N*-(4-((7-ethoxy-6-methoxyquinazolin-4-yl)oxy)-3-fluorophenyl)-1,2-dimethyl-4-oxo-6-(triflu oromethoxy)-1,4-dihydroquinoline-3-carboxamide,
*N*-(3-fluoro-4-((7-methoxy-6-(3-morpholinopropoxy)quinazolin-4-yl)oxy)phenyl)-1,2-dimethy l-4-oxo-6-(trifluoromethoxy)-1,4-dihydroquinoline-3-carboxamide,
and
*N*-(3-fluoro-4-((6-methoxy-7-(3-morpholinopropoxy)quinolin-4-yl)oxy)phenyl)-1,2-dimethyl-4-oxo -6-(trifluoromethoxy)-1,4-dihydroquinoline-3-carboxamide.

**9.** A pharmaceutical composition comprising an active ingredient and a pharmaceutically acceptable excipient, wherein the active ingredient comprises the quinoline or quinazoline compound of any one of claims 1 to 8 or a pharmaceutically acceptable salt thereof, a stereoisomer thereof, or a deuterated analog thereof.

**10.** A quinoline or quinazoline compound of any one of claims 1 to 8 or a pharmaceutically acceptable salt thereof, a stereoisomer thereof, or a deuterated analog thereof or the pharmaceutical composition of claim 9 for use in preventing or treating a tumor.

**11.** The quinoline or quinazoline compound or a pharmaceutically acceptable salt thereof, a stereoisomer thereof, or a deuterated analog thereof or the pharmaceutical composition for use according to claim 10, wherein the tumor is hematological tumor, gastrointestinal stromal tumor, histiocytic lymphoma, non-small cell lung cancer, small cell lung cancer, lung adenocarcinoma, lung squamous cell carcinoma, pancreatic cancer, breast cancer, prostate cancer, liver cancer, skin cancer, epithelial cell carcinoma, or nasopharyngeal carcinoma.

## Patentansprüche

**1.** Chinolin- oder Chinazolinverbindung mit einer Struktur, die dargestellt ist durch eine Formel (I), oder ein pharmazeutisch annehmbares Salz davon, ein Stereoisomer davon oder ein deuteriertes Analogon davon:

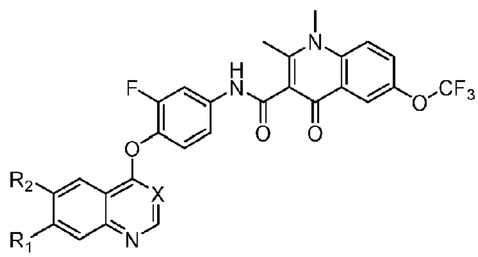

(I)

wobei X ausgewählt ist aus CH und N;

$R_1$ und $R_2$ jeweils unabhängig ausgewählt sind aus einer Gruppe bestehend aus Wasserstoff, Halogen, $-(CR_4R_5)_oR_3$ und $-O(CR_4R_5)_oR_3$;
wobei o eine ganze Zahl von 0 bis 6 ist;
$R_3$, $R_4$ und $R_5$ jeweils unabhängig ausgewählt sind aus einer Gruppe bestehend aus -H, $C_1$~$C_6$-Alkyl, Halogen, $-CF_3$, $-OCF_3$, $-(C{=}O)-NR_8R_9$, $-COOR_8$, $-SO_m-NR_8R_9$, $-CHR_8R_9$, $-OR_8$ und $-NR_8R_9$;
$R_8$ und $R_9$ jeweils unabhängig ausgewählt sind aus einer Gruppe bestehend aus Wasserstoff, Halogen und $C_1$~$C_6$-Alkyl, oder $R_8$ und $R_9$ zusammen mit daran gebundenem N eine gesättigte oder ungesättigte 5- bis 8-gliedrige heterocyclische Gruppe bilden; wobei die gesättigte oder ungesättigte 5- bis 8-gliedrige heterocyclische Gruppe unabhängig und optional mit einem oder mehreren $R_{10}$ substituiert sein kann; wobei $R_{10}$ ausgewählt ist aus einer Gruppe bestehend aus $C_1$~$C_6$-Alkyl;
oder $R_1$ und $R_2$ ein substituiertes oder unsubstituiertes aliphatisches $C_5$~$C_{18}$-Cycloalkyl bilden, das 1 bis 4 Heteroatome enthält.

**2.** Chinolin- oder Chinazolinverbindung nach Anspruch 1 oder ein pharmazeutisch annehmbares Salz davon, ein Stereoisomer davon oder ein deuteriertes Analogon davon, wobei $R_1$ und $R_2$ jeweils unabhängig für $-O(CR_4R_5)_oR_3$ stehen; $R_3$, $R_4$ und $R_5$ jeweils unabhängig ausgewählt sind aus einer Gruppe bestehend aus -H, $C_1$~$C_6$-Alkyl, $-OR_8$ und $-NR_8R_9$; $R_8$ und $R_9$ jeweils unabhängig ausgewählt sind aus einer Gruppe bestehend aus $C_1$~$C_6$-Alkyl oder $R_8$ und $R_9$ zusammen mit daran gebundenem N eine gesättigte oder ungesättigte 5- bis 8-gliedrige heterocyclische Gruppe bilden; wobei die gesättigte oder ungesättigte 5- bis 8-gliedrige heterocyclische Gruppe unabhängig und optional durch ein oder mehrere $R_{10}$ substituiert sein kann; wobei $R_{10}$ ausgewählt ist aus einer Gruppe bestehend aus $C_1$~$C_6$-Alkyl.

**3.** Chinolin- oder Chinazolinverbindung nach Anspruch 2 oder ein pharmazeutisch annehmbares Salz davon, ein Stereoisomer davon oder ein deuteriertes Analogon davon, wobei $R_1$ $-O(CH_2)_oR_3$ ist;

o eine ganze Zahl von 0 bis 4 ist; $R_3$ ausgewählt ist aus einer Gruppe bestehend aus -H, $C_1$~$C_6$-Alkyl, $C_1$~$C_3$-Alkoxy und $-NR_8R_9$;
$R_8$ und $R_9$ jeweils unabhängig ausgewählt sind aus einer Gruppe bestehend aus $C_1$~$C_3$-Alkyl, oder $R_8$ und $R_9$ zusammen mit daran gebundenem N eine gesättigte oder ungesättigte 5- bis 6-gliedrige heterocyclische Gruppe bilden; wobei die gesättigte oder ungesättigte 5- bis 6-gliedrige heterocyclische Gruppe unabhängig und optional durch ein oder mehrere $R_{10}$ substituiert sein kann; wobei $R_{10}$ ausgewählt ist aus einer Gruppe bestehend aus $C_1$~$C_3$-Alkyl.

**4.** Chinolin- oder Chinazolinverbindung nach Anspruch 3 oder ein pharmazeutisch annehmbares Salz davon, ein Stereoisomer davon oder ein deuteriertes Analogon davon, wobei $R_1$ ausgewählt ist aus einer Gruppe bestehend aus Methoxyl, Ethoxyl, Propoxyl, 2-Methoxyethoxyl, 3-Methoxypropoxyl, 3-Morpholinopropoxyl, 2-(Pyrrolidin-1-yl)ethoxyl, 3-(Pyrrolidin-1-yl)propoxyl, (Piperidin-1-yl)ethoxyl, (Piperidin-1-yl)propoxyl, 4-Methoxybutoxyl, 2-Morpholinoethoxyl, (4-Methylpiperazin-1-yl)propoxyl, Dimethylaminoethoxyl und Isopentyloxyl.

**5.** Chinolin- oder Chinazolinverbindung nach Anspruch 2 oder ein pharmazeutisch annehmbares Salz davon, ein Stereoisomer davon oder ein deuteriertes Analogon davon, wobei $R_2$ $-O(CH_2)_oR_3$ ist;

o eine ganze Zahl von 0 bis 4 ist;
$R_3$ ausgewählt ist aus einer Gruppe bestehend aus: -H, $C_1$~$C_3$-Alkyl, $C_1$~$C_3$-Alkoxy und $-NR_8R_9$;
$R_8$ und $R_9$ zusammen mit daran gebundenem N eine gesättigte 5- bis 6-gliedrige heterocyclische Gruppe bilden.

**6.** Chinolin- oder Chinazolinverbindung nach Anspruch 5 oder ein pharmazeutisch annehmbares Salz davon, ein Stereoisomer davon oder ein deuteriertes Analogon davon, wobei $R_2$ ausgewählt ist aus einer Gruppe bestehend aus Methoxyl, Ethoxyl, Propoxyl, 2-Methoxyethoxyl, 3-Methoxypropoxyl, 2-Morpholinoethoxyl und 3-Morpholinopropoxyl.

**7.** Chinolin- oder Chinazolinverbindung nach einem der Ansprüche 1 bis 6 oder ein pharmazeutisch annehmbares Salz davon, ein Stereoisomer davon oder ein deuteriertes Analogon davon, wobei X N ist.

**8.** Chinolin- oder Chinazolinverbindung nach Anspruch 1 oder ein pharmazeutisch annehmbares Salz davon, ein Stereoisomer davon oder ein deuteriertes Analogon davon, wobei die Chinolin- oder Chinazolinverbindung ausgewählt ist aus einer Gruppe bestehend aus:

*N*-(3-Fluor-4-((6-methoxy-7-(3-morpholinopropoxy)chinazolin-4-yl)oxy)phenyl)-1,2-dimethyl-4-oxo-6-(trifluormethoxy)-1,4-dihydrochinolin-3-carboxamid,
*N*-(4-((6,7-Bis(2-methoxyethoxy)chinazolin-4-yl)oxy)-3-fluorphenyl)-1,2-dimethyl-4-oxo-6-(trifluormethoxy)-1,4-dihydrochinolin-3-carboxamid,
*N*-(4-((6,7-Dimethoxychinazolin-4-yl)oxy)-3-fluorphenyl)-1,2-dimethyl-4-oxo-6-(trifluormethoxy)-1,4-dihydrochinolin-3-carboxamid,
*N*-(3-Fluor-4-((7-methoxy-6-(3-methoxypropoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(4-methoxybutoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(2-morpholinoethoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(3-(pyrrolidin-1-yl)propoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(2-(piperidin-1-yl)ethoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(3-(4-methylpiperazin-1-yl)propoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(2-(pyrrolidin-1-yl)ethoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(3-methoxypropoxy)chinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-(3-(piperidin-1-yl)propoxy)chinazolin-4-yl)oxy)phenyl)-1,2-dimethyl-4-oxo-6-(trifluormethoxy)-1,4-dihydrochinolin-3-carboxamid,
*N*-(4-((7-(2-(Dimethylamino)ethoxy)-6-methoxychinazolin-4-yl)oxy)-3-fluorphenyl)-1,2-dimethyl-4-oxo-6-(trifluormethoxy)-1,4-dihydrochinolin-3-carboxamid,
*N*-(3-Fluor-4-((7-(isopentyloxy)-6-methoxychinazolin-4-yl)oxy)phenyl)-1,2-dimethyl-4-oxo-6-(trifluormethoxy)-1,4-dihydrochinolin-3-carboxamid,
*N*-(3-Fluor-4-((6-methoxy-7-propoxychinazolin-4-yl)oxy)phenyl)-1,2-dimethyl-4-oxo-6-(trifluormethoxy)-1,4-dihydrochinolin-3-carboxamid,
*N*-(4-((7-Ethoxy-6-methoxychinazolin-4-yl)oxy)-3-fluorphenyl)-1,2-dimethyl-4-oxo-6-(trifluormethoxy)-1,4-dihydrochinolin-3-carboxamid,
*N*-(3-Fluor-4-((7-methoxy-6-(3-morpholinopropoxy)chinazolin-4-yl)oxy)phenyl)-1,2-dimethyl-4-oxo-6-(trifluormethoxy)-1,4-dihydrochinolin-3-carboxamid
und
*N*-(3-Fluor-4-((6-methoxy-7-(3-morpholinopropoxy)chinolin-4-yl)oxy)phenyl)-1,2-dimethyl-4-oxo-6-(trifluormethoxy)-1,4-dihydrochinolin-3-carboxamid.

**9.** Pharmazeutische Zusammensetzung, umfassend einen Wirkstoff und einen pharmazeutisch annehmbaren Hilfsstoff, wobei der Wirkstoff die Chinolin- oder Chinazolinverbindung nach einem der Ansprüche 1 bis 8 oder ein pharmazeutisch annehmbares Salz davon, ein Stereoisomer davon oder ein deuteriertes Analogon davon umfasst.

**10.** Chinolin- oder Chinazolinverbindung nach einem der Ansprüche 1 bis 8 oder ein pharmazeutisch annehmbares Salz davon, ein Stereoisomer davon oder ein deuteriertes Analogon davon oder pharmazeutische Zusammensetzung nach Anspruch 9 zur Verwendung bei der Prävention oder Behandlung eines Tumors.

**11.** Chinolin- oder Chinazolinverbindung oder ein pharmazeutisch annehmbares Salz davon, ein Stereoisomer davon oder ein deuteriertes Analogon davon oder pharmazeutische Zusammensetzung zur Verwendung nach Anspruch 10, wobei es sich bei dem Tumor um einen hämatologischen Tumor, gastrointestinalen Stromatumor, histiozytäres Lymphom, nicht-kleinzelligen Lungenkrebs, kleinzelligen Lungenkrebs, Lungenadenokarzinom, Lungenplattenepithelkarzinom, Bauchspeicheldrüsenkrebs, Brustkrebs, Prostatakrebs, Leberkrebs, Hautkrebs, Epithelkarzinom oder Nasopharynxkarzinom handelt.

## Revendications

**1.** Composé de quinoléine ou de quinazoline ayant une structure représentée par une formule (I) ou sel pharmaceutiquement acceptable de celui-ci, stéréoisomère de celui-ci ou analogue deutéré de celui-ci :

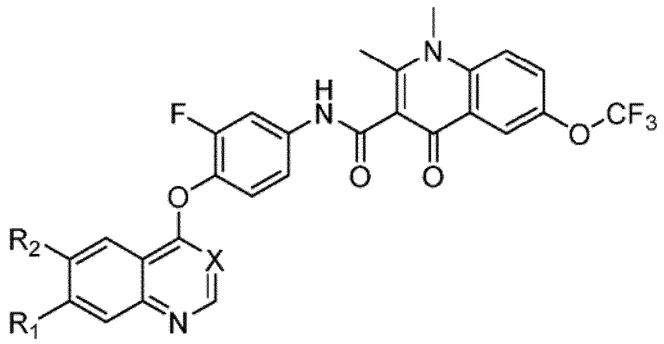

(I)

dans lequel, X est choisi parmi CH et N ;

$R_1$ et $R_2$ sont chacun indépendamment choisis dans un groupe constitué d'hydrogène, halogène, $-(CR_4R_5)oR_3$ et $-O(CR_4R_5)oR_3$ ;
dans lequel, o est un entier de 0 à 6 ;
$R_3$, $R_4$ et $R_5$ sont chacun indépendamment choisis dans un groupe constitué de -H, alkyle en $C_1$~$C_6$, halogène, $-CF_3$, $-OCF_3$, $-(C=O)-NR_8R_9$, $-COOR_8$, $-SO_m-NR_8R_9$, $-CHR_8R_9$, $-OR_8$ et $-NR_8R_9$ ;
$R_8$ et $R_9$ sont chacun indépendamment choisis dans un groupe constitué d'hydrogène, halogène et alkyle en $C_1$~$C_6$, ou, $R_8$ et Rg, conjointement avec N relié à ceux-ci, forment un groupe hétérocyclique de 5 à 8 chaînons saturé ou insaturé ; dans lequel, le groupe hétérocyclique de 5 à 8 chaînons saturé ou insaturé peut être indépendamment et facultativement substitué par un ou plusieurs $R_{10}$ ; dans lequel $R_{10}$ est choisi dans un groupe constitué d'alkyle en $C_1$~$C_6$ ;
ou, $R_1$ et $R_2$ forment un cycloalkyle aliphatique en $C_5$~$C_{18}$ substitué ou non substitué contenant 1 à 4 hétéroatomes.

**2.** Composé de quinoléine ou de quinazoline selon la revendication 1 ou sel pharmaceutiquement acceptable de celui-ci, stéréoisomère de celui-ci ou analogue deutéré de celui-ci, dans lequel $R_1$ et $R_2$ sont chacun indépendamment $-O(CR_4R_5)oR_3$ ;
$R_3$, $R_4$ et $R_5$ sont chacun indépendamment choisis dans un groupe constitué de -H, alkyle en $C_1$~$C_6$, $-OR_8$ et $-NR_8R_9$ ; $R_8$ et $R_9$ sont chacun indépendamment choisis dans un groupe constitué d'alkyle en $C_1$~$C_6$, ou, $R_8$ et Rg, conjointement avec N relié à ceux-ci, forment un groupe hétérocyclique de 5 à 8 chaînons saturé ou insaturé ; dans lequel, le groupe hétérocyclique de 5 à 8 chaînons saturé ou insaturé peut être indépendamment et facultativement substitué par un ou plusieurs $R_{10}$ ; dans lequel $R_{10}$ est choisi dans un groupe constitué d'alkyle en $C_1$~$C_6$.

**3.** Composé de quinoléine ou de quinazoline selon la revendication 2 ou sel pharmaceutiquement acceptable de celui-ci, stéréoisomère de celui-ci ou analogue deutéré de celui-ci, dans lequel $R_1$ est $-O(CH_2)oR_3$ ;

o est un entier de 0 à 4 ; $R_3$ est choisi dans un groupe constitué de -H, alkyle en $C_1$~$C_6$, alcoxy en $C_1$~$C_3$ et $-NR_8R_9$ ;
$R_8$ et $R_9$ sont chacun indépendamment choisis dans un groupe constitué d'alkyle en $C_1$~$C_3$, ou $R_8$ et Rg, conjointement avec N relié à ceux-ci, forment un groupe hétérocyclique de 5 à 6 chaînons saturé ou insaturé ; dans lequel, le groupe hétérocyclique de 5 à 6 chaînons saturé ou insaturé peut être indépendamment et facultativement substitué par un ou plusieurs $R_{10}$ ; dans lequel $R_{10}$ est choisi dans un groupe constitué d'alkyle en $C_1$~$C_3$.

**4.** Composé de quinoléine ou de quinazoline selon la revendication 3 ou sel pharmaceutiquement acceptable de celui-ci, stéréoisomère de celui-ci ou analogue deutéré de celui-ci, dans lequel $R_1$ est choisi dans un groupe constitué de méthoxyle, éthoxyle, propoxyle, 2-méthoxyéthoxyle, 3-méthoxypropoxyle, 3-morpholinopropoxyle, 2-(pyrrolidin-1-yl)éthoxyle, 3-(pyrrolidin-1-yl)propoxyle, (pipéridin-1-yl)éthoxyle, (pipéridin-1-yl)propoxyle, 4-méthoxybutoxyle, 2-morpholinoéthoxyle, (4-méthylpipérazin-1-yl)propoxyle, diméthylaminoéthoxyle et isopentyloxyle.

**5.** Composé de quinoléine ou de quinazoline selon la revendication 2 ou sel pharmaceutiquement acceptable de celui-ci, stéréoisomère de celui-ci ou analogue deutéré de celui-ci, dans lequel $R_2$ est $-O(CH_2)oR_3$ ;

o est un entier de 0 à 4 ;
$R_3$ est choisi dans un groupe constitué de : -H, alkyle en $C_1$~$C_3$, alcoxy en $C_1$~$C_3$ et $-NR_8R_9$ ;
$R_8$ et Rg, conjointement avec N relié à ceux-ci, forment un groupe hétérocyclique de 5 à 6 chaînons saturé.

**6.** Composé de quinoléine ou de quinazoline selon la revendication 5 ou sel pharmaceutiquement acceptable de celui-ci, stéréoisomère de celui-ci ou analogue deutéré de celui-ci, dans lequel $R_2$ est choisi dans un groupe constitué de méthoxyle, éthoxyle, propoxyle, 2-méthoxyéthoxyle, 3-méthoxypropoxyle, 2-morpholinoéthoxyle et 3-morpholinopropoxyle.

**7.** Composé de quinoléine ou de quinazoline selon l'une quelconque des revendications 1 à 6 ou sel pharmaceutiquement acceptable de celui-ci, stéréoisomère de celui-ci ou analogue deutéré de celui-ci, dans lequel X est N.

**8.** Composé de quinoléine ou de quinazoline selon la revendication 1 ou sel pharmaceutiquement acceptable de celui-ci, stéréoisomère de celui-ci ou analogue deutéré de celui-ci, le composé de quinoléine ou de quinazoline étant choisi dans un groupe constitué de :

*N*-(3-fluoro-4-((6-méthoxy-7-(3-morpholinopropoxy)quinazolin-4-yl)oxy)phényl)-1,2-diméthyl-4-oxo-6-(trifluorométhoxy)-1,4-dihydroquinoléine-3-carboxamide,
*N*-(4-((6,7-bis(2-méthoxyéthoxy)quinazolin-4-yl)oxy)-3-fluorophényl)-1,2-diméthyl-4-oxo-6-(trifluorométhoxy)-1,4-dihydroquinoléine-3-carboxamide,
*N*-(4-((6,7-diméthoxyquinazolin-4-yl)oxy)-3-fluorophényl)-1,2-diméthyl-4-oxo-6-(trifluorométhoxy)-1,4-dihydroquinoléine-3-carboxamide,
*N*-(3-fluoro-4-((7-méthoxy-6-(3-méthoxypropoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(4-méthoxybutoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(2-morpholinoéthoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(3-(pyrrolidin-1-yl)propoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(2-(pipéridin-1-yl)éthoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(3-(4-méthylpipérazin-1-yl)propoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(2-(pyrrolidin-1-yl)éthoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(3-méthoxypropoxy)quinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-(3-(pipéridin-1-yl)propoxy)quinazolin-4-yl)oxy)phényl)-1,2-diméthyl-4-oxo-6-(trifluorométhoxy)-1,4-dihydroquinoléine-3-carboxamide,
*N*-(4-((7-(2-(diméthylamino)éthoxy)-6-méthoxyquinazolin-4-yl)oxy)-3-fluorophényl)-1,2-diméthyl-4-oxo-6-(trifluorométhoxy)-1,4-dihydroquinoléine-3-carboxamide,
*N*-(3-fluoro-4-((7-(isopentyloxy)-6-méthoxyquinazolin-4-yl)oxy)phényl)-1,2-diméthyl-4-oxo-6-(trifluorométhoxy)-1,4-dihydroquinoléine-3-carboxamide,
*N*-(3-fluoro-4-((6-méthoxy-7-propoxyquinazolin-4-yl)oxy)phényl)-1,2-diméthyl-4-oxo-6-(trifluorométhoxy)-1,4-dihydroquinoléine-3-carboxamide,
*N*-(4-((7-éthoxy-6-méthoxyquinazolin-4-yl)oxy)-3-fluorophényl)-1,2-diméthyl-4-oxo-6-(trifluorométhoxy)-1,4-dihydroquinoléine-3-carboxamide,
*N*-(3-fluoro-4-((7-méthoxy-6-(3-morpholinopropoxy)quinazolin-4-yl)oxy)phényl)-1,2-diméthyl-4-oxo-6-(trifluorométhoxy)-1,4-dihydroquinoléine-3-carboxamide,
et
*N*-(3-fluoro-4-((6-méthoxy-7-(3-morpholinopropoxy)quinoléin-4-yl)oxy)phényl)-1,2-diméthyl-4-oxo-6-(trifluorométhoxy)-1,4-dihydroquinoléine-3-carboxamide.

**9.** Composition pharmaceutique comprenant une substance active et un excipient pharmaceutiquement acceptable, dans laquelle la substance active comprend le composé de quinoléine ou de quinazoline selon l'une quelconque des revendications 1 à 8 ou un sel pharmaceutiquement acceptable de celui-ci, un stéréoisomère de celui-ci ou un analogue deutéré de celui-ci.

**10.** Composé de quinoléine ou de quinazoline selon l'une quelconque des revendications 1 à 8 ou sel pharmaceutiquement acceptable de celui-ci, stéréoisomère de celui-ci ou analogue deutéré de celui-ci ou la composition pharmaceutique selon la revendication 9 pour utilisation dans la prévention ou le traitement d'une tumeur.

**11.** Composé de quinoléine ou de quinazoline ou sel pharmaceutiquement acceptable de celui-ci, stéréoisomère de celui-ci ou analogue deutéré de celui-ci ou composition pharmaceutique pour utilisation selon la revendication 10, la tumeur étant une tumeur hématologique, une tumeur stromale gastro-intestinale, un lymphome histiocytaire, un cancer du poumon non à petites cellules, un cancer du poumon à petites cellules, un adénocarcinome pulmonaire, un carcinome épidermoïde pulmonaire, un cancer du pancréas, un cancer du sein, un cancer de la prostate, un cancer du foie, un cancer de la peau, un carcinome à cellules épithéliales ou un carcinome du nasopharynx.

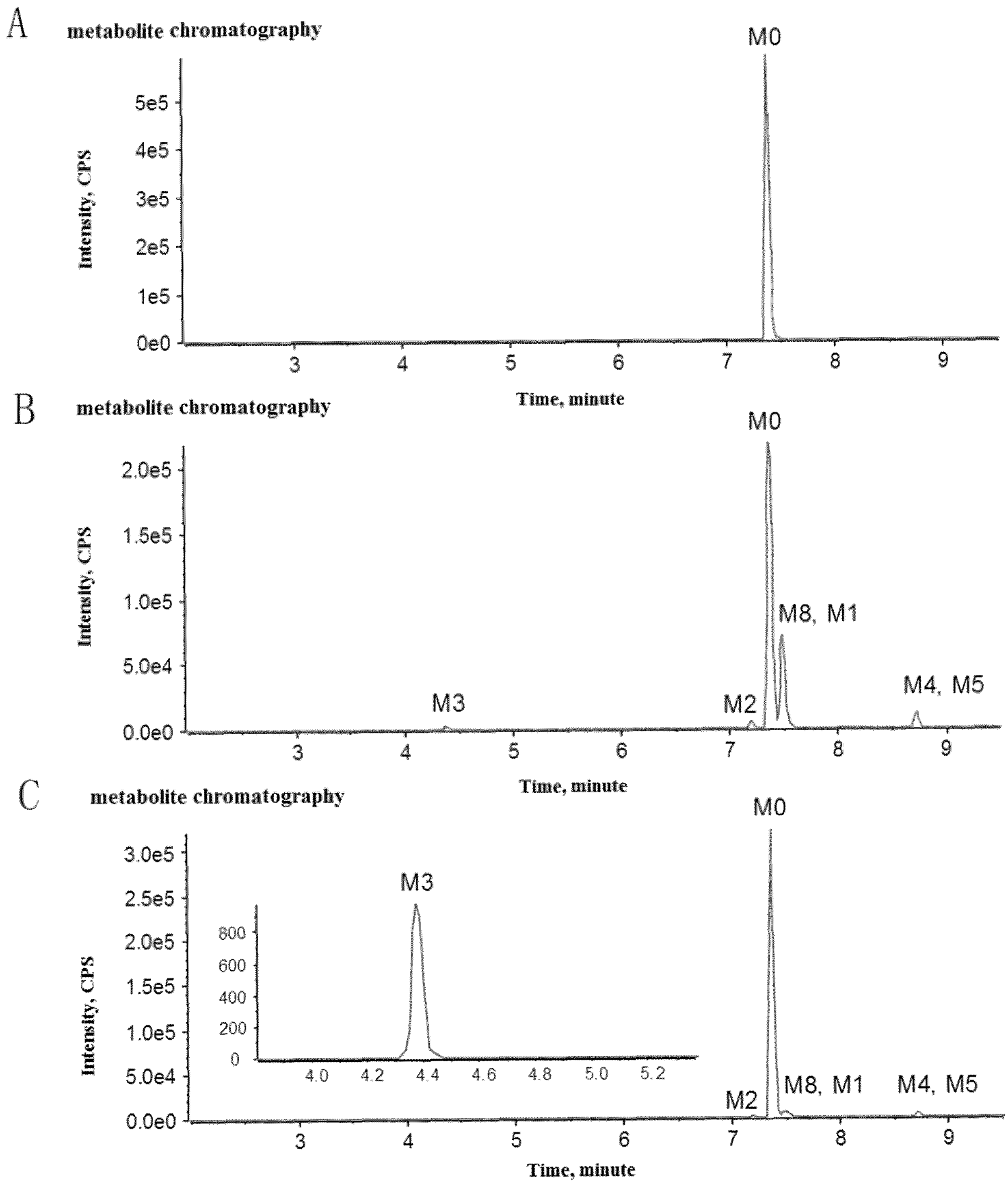
A
metabolite chromatography
M0
Intensity, CPS
5e5
4e5
3e5
2e5
1e5
0e0
3
4
5
6
7
8
9
Time, minute
B
metabolite chromatography
M0
Intensity, CPS
2.0e5
1.5e5
1.0e5
5.0e4
0.0e0
M8, M1
M4, M5
M3
M2
Time, minute
C
metabolite chromatography
M0
Intensity, CPS
3.0e5
2.5e5
2.0e5
1.5e5
1.0e5
5.0e4
0.0e0
M3
800
600
400
200
0
4.0
4.2
4.4
4.6
4.8
5.0
5.2
M8, M1
M4, M5
M2
Time, minute

FIG.1

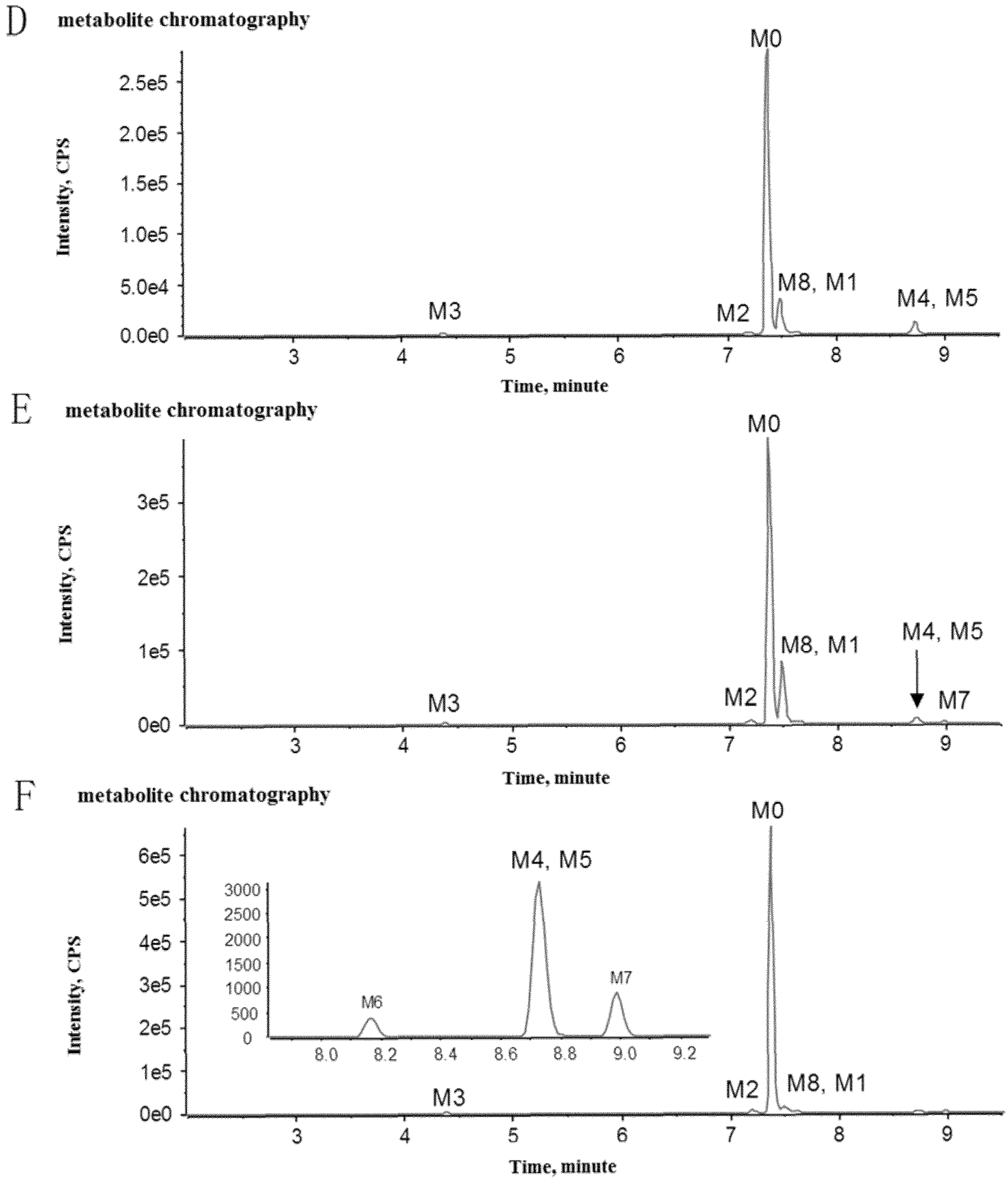
D
metabolite chromatography
Intensity, CPS
2.5e5
2.0e5
1.5e5
1.0e5
5.0e4
0.0e0
M0
M8, M1
M4, M5
M3
M2
3
4
5
6
7
8
9
Time, minute
E
metabolite chromatography
Intensity, CPS
3e5
2e5
1e5
0e0
M0
M8, M1
M4, M5
M7
M3
M2
3
4
5
6
7
8
9
Time, minute
F
metabolite chromatography
Intensity, CPS
6e5
5e5
4e5
3e5
2e5
1e5
0e0
M0
M4, M5
3000
2500
2000
1500
1000
500
0
M6
M7
8.0
8.2
8.4
8.6
8.8
9.0
9.2
M3
M2
M8, M1
3
4
5
6
7
8
9
Time, minute

FIG.2

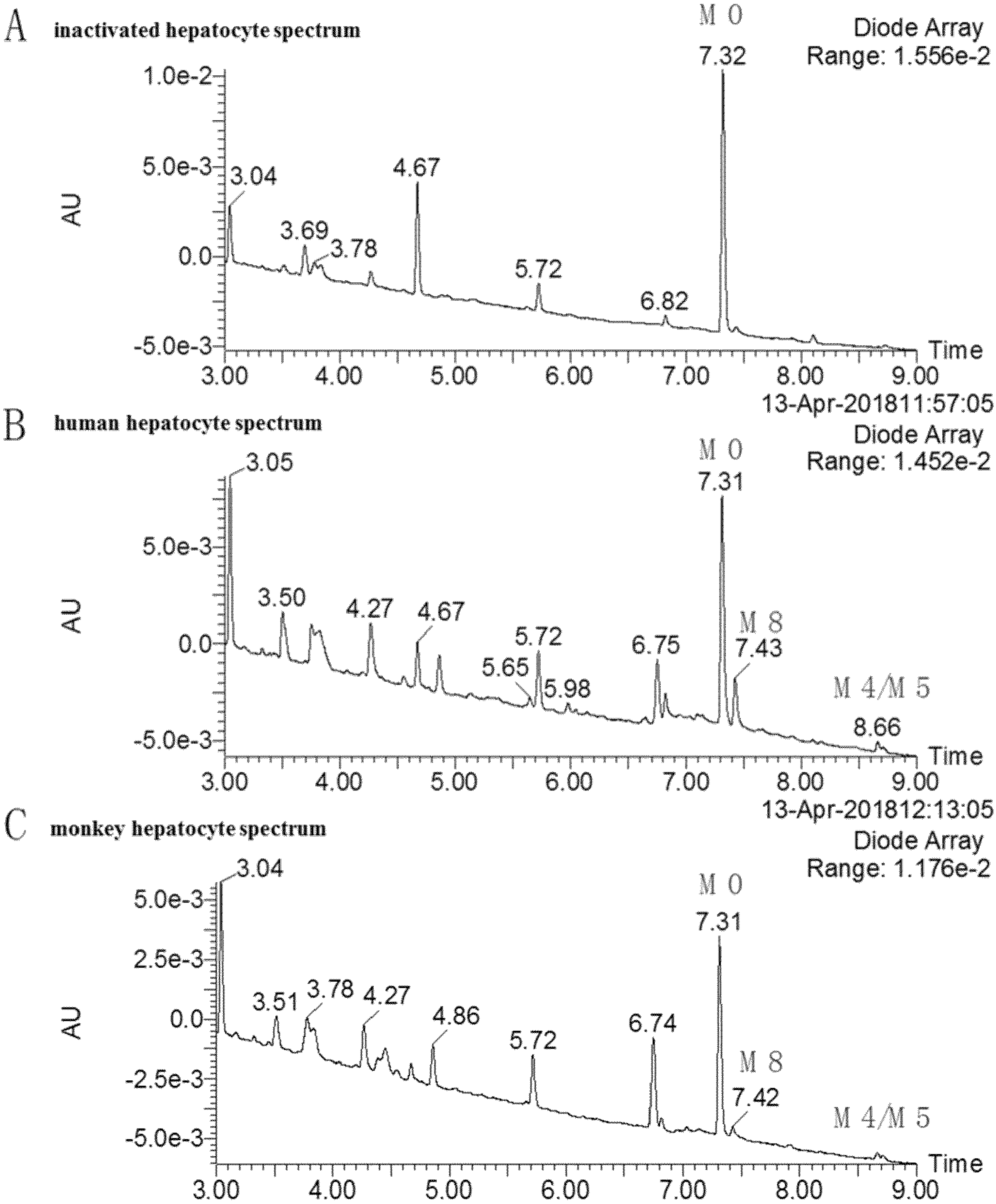
A inactivated hepatocyte spectrum
M 0
7.32
Diode Array
Range: 1.556e-2
1.0e-2
5.0e-3
0.0
-5.0e-3
AU
3.04
3.69
3.78
4.67
5.72
6.82
Time
3.00 4.00 5.00 6.00 7.00 8.00 9.00
13-Apr-201811:57:05
B human hepatocyte spectrum
Diode Array
Range: 1.452e-2
M 0
7.31
3.05
5.0e-3
0.0
-5.0e-3
AU
3.50
4.27
4.67
5.72
5.65
5.98
6.75
M 8
7.43
M 4/M 5
8.66
Time
3.00 4.00 5.00 6.00 7.00 8.00 9.00
13-Apr-201812:13:05
C monkey hepatocyte spectrum
Diode Array
Range: 1.176e-2
3.04
M 0
7.31
5.0e-3
2.5e-3
0.0
-2.5e-3
-5.0e-3
AU
3.51
3.78
4.27
4.86
5.72
6.74
M 8
7.42
M 4/M 5
Time
3.00 4.00 5.00 6.00 7.00 8.00 9.00

FIG.3

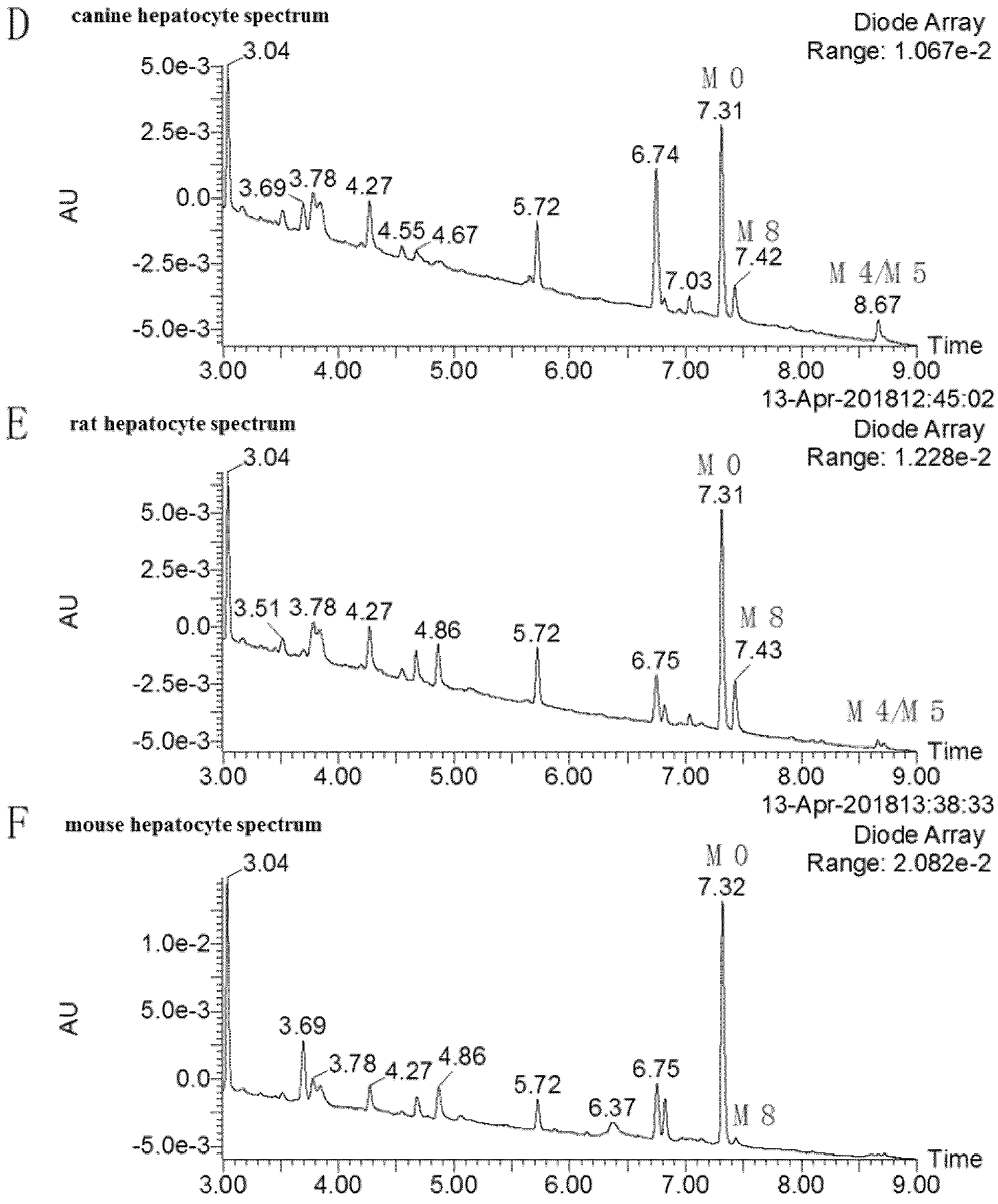
D
canine hepatocyte spectrum
Diode Array
Range: 1.067e-2
3.04
M 0
7.31
6.74
3.69
3.78
4.27
5.72
4.55
4.67
M 8
7.42
7.03
M 4/M 5
8.67
AU
Time
13-Apr-201812:45:02
E
rat hepatocyte spectrum
Diode Array
Range: 1.228e-2
3.04
M 0
7.31
3.51
3.78
4.27
4.86
5.72
6.75
M 8
7.43
M 4/M 5
AU
Time
13-Apr-201813:38:33
F
mouse hepatocyte spectrum
Diode Array
Range: 2.082e-2
3.04
M 0
7.32
3.69
3.78
4.27
4.86
5.72
6.37
6.75
M 8
AU
Time

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017028797 A


### Non-patent literature cited in the description


- **BERG et al.** *Pharmaceutical Salts, J. Pharm. Sci.,* 1977, vol. 66, 1-19